# EUROPEAN PATENT SPECIFICATION

(11) **EP 4 559 417 B1**
(45) Date of publication and mention of the grant of the patent: **19.08.2026**
(21) Application number: 23843405.4
(22) Date of filing: 20.07.2023
(51) Int. Cl.: A61B 17/42, A61B 17/02, A61B 34/30, A61B 34/00, A61B 17/34

(54) **UTERINE MANIPULATOR, UTERINE MANIPULATOR ROBOT COMPRISING SAME, AND UTERINE MANIPULATOR SYSTEM COMPRISING SAME**
UTERUSMANIPULATOR, UTERUSMANIPULATORROBOTER DAMIT UND UTERUSMANIPULATORSYSTEM DAMIT
MANIPULATEUR UTÉRIN, ROBOT MANIPULATEUR UTÉRIN LE COMPRENANT, ET SYSTÈME MANIPULATEUR UTÉRIN LE COMPRENANT

(30) Priority: 20.07.2022 KR 20220089564
(43) Date of publication of application: 28.05.2025
(73) Proprietor: Ezmedibot Co., Ltd., Seongnam-si, Gyeonggi-do 13219 (KR)
(72) Inventor: KIM, Byung-kyu, Seoul 03911 (KR); KIM, Soo-young, Goyang-si Gyeonggi-do 10476 (KR); HWANG, Bo-hyun, Incheon 22697 (KR); KANG, Hyung-seok, Seoul 02082 (KR); CHUNG, Hyun-hoon, Seoul 06084 (KR); KIM, Se-ik, Seoul 06587 (KR)
(74) Representative: dompatent
(86) International application number: PCT/KR2023/010474
(87) International publication number: WO 2024/019559

(56) References cited:
- WO-A1-2021/225863
- WO-A1-96/03930
- CN-B- 107 019 548
- JP-A- 2011 004 880
- KR-A- 20190 045 888
- KR-A- 20210 085 037
- US-A1- 2009 326 325
- US-A1- 2021 228 238
- US-A1- 2021 282 814

## Description

### [TECHNICAL FIELD]

The present disclosure relates to a uterine manipulator, a uterine manipulator robot including the same, and a uterine manipulator system including the same.

This research was supported by research funds for a project (Research period: January 1, 2022 to December 31, 2023) of "Development of Microrobot for Precisely Injecting of Cancer Cell-specific Nanobots and In-vitro Assistance Robots" of a development and research business for commercialization of micro-medical robot products (Project ID number: 1465037017, project number: HI19C0664010222, Ministry name: Ministry of Health and Welfare, Project management (professional) agency name: Korea Health Industry Promotion Agency, Project implementation agency: Korea Aerospace University Industry-Academic Cooperation Group).

### [BACKGROUND ART]

A laparoscopic gynecological surgery (a laparoscopic hysterectomy, or the like) using a minimally invasive surgery has difficulty obtaining a visibility in parts of the uterus due to the limitations of physically manipulating of the laparoscope, and to assist this, an uterine manipulator that is a device that may manipulate a position of a uterus as a portion of the device is inserted into a vagina to be moved is additionally used.

To proceed with conventional laparoscopic surgery, not only a main surgeon but also an assistant who may operate a uterine manipulator are required, and because the manipulation is performed entirely through communication with the surgeon, the precision becomes lower, the safety deteriorates, and the surgery time is delayed.

According to an automated intrauterine manipulation robot system (Da Vinci, or the like) used in clinical practices, the movement of the robot is not ergonomic, and thus, an organ of the patient may be damaged, the operation method is not intuitive, and thus, it takes time for the operator to become proficient in the operation, and the surgery safety is low because the surgical operator cannot predict changes in the position or orientation of the uterus.

The background technology of the present disclosure is disclosed in Korean Patent Application No. 10-2021-0042332 (the name of the invention "Uterine Manipulator"). This existing patent technology is a robot system that controls the position of the uterus with a three degree of freedom (a pitch, a yaw, and a translation), and a distal end of the robot (i.e. end effector) is inserted directly into the vagina and fixed to the uterus and the cervix, but their sizes make it difficult to install them in the human body. In addition, according to the existing patent technology, a drug is injected depending on the sense of the surgical operator.

In addition, the technology that is the background technology is disclosed in U.S. Patent Application Publication No. 2020-0253676 (the name of the invention "Robotically-operated Uterine Manipulators"). According to the conventional uterine manipulator (US5409469) for hysterectomy, because the position of the uterus is manipulated through the manipulation of the assistant, and thus, an accurate position control is difficult, and obstacles to the progress of the surgery occur due to communication problems with the main surgeon. According to the existing patent technologies, a workspace outside the patient may be manipulated, and because it sometimes requires an excessive movement to manipulate the uterus, the uterus may be damaged during the manipulation. Further background art is disclosed in WO 2021/225863 A1, which describes a medical instrument with a wrist assembly actuated by a plurality of cables. The wrist assembly includes multiple links that are rotatably coupled to produce pitch and yaw movements. The cables are driven by a proximal mechanical structure including capstan assemblies, where a single capstan can move multiple cables at different speeds by using cable drums of different radii

### [DETAILED DESCRIPTION OF THE INVENTION]

### [TECHNICAL PROBLEM]

The invention is defined in the appended set of claims. The present disclosure has been made to solve the problems of the above-described conventional technology, and provides a uterine manipulator, a uterine manipulator robot including the same, and a uterine manipulator system including the same, by which a safety and a stability of a surgery may be obtained by directly intuitively adjusting a position and a posture of a uterus by a surgical operator.

The technical problem to be solved by embodiments of the present disclosure is not limited to the above-described technical problems, and other technical problems may be deduced.

### [TECHNICAL SOLUTION]

According to an aspect of the present disclosure, a uterine manipulator includes an operation module including an end tip, and an operation structure including an end member provided with the operation module at a front end thereof, and a pitch operation part allowing a pitch operation of rotating the end member about a first axis such that a pitch in a direction which the end tip faces is adjusted, and the pitch operation part includes a first joint disposed to be rotatable while an upward/downward direction is taken as a rotation axis thereof, a second joint disposed on a front side of the first joint, and disposed to be rotatable while the upward/downward direction is taken as a rotation axis thereof, a first connection part that rotates the second joint by transmitting a rotational force of the first joint to the second joint when the first joint is rotated, a third joint disposed on a front side of the second joint, provided with the end member at a front end thereof, and disposed to be rotatable while a leftward/rightward direction is taken as a rotation axis thereof, and a second connection part that rotates the third joint by transmitting a rotational force of the second joint to the third joint when the second joint is rotated.

According to an aspect of the present disclosure, a uterine manipulator robot includes the uterine manipulator, and a driving part that drives the operation structure, the uterine manipulator further includes a handle part connected to the rotary shaft of the first joint, and that manipulates rotation of the first joint about a rotation axis thereof, and the driving part includes a seating part, on which a rear end of the uterine manipulator is seated, and an automatic pitch manipulation part that rotates the handle part seated on the seating part.

According to an aspect of the present disclosure, a uterine manipulator system includes the uterine manipulator robot, and an additional pitch operation part allowing a pitch operation of rotating the robot about the first axis such that a pitch in a direction which the end tip faces is adjusted.

The above-described problem solving means are illustrative only and should not be construed with the intention of limiting the present application. In addition to the above-described embodiment, additional embodiments may exist in the drawings and the detailed description of the present invention.

### [ADVANTAGEOUS EFFECTS OF THE INVENTION]

According to the problem solving means of the present disclosure described above, because the operation module may perform a pitch operation with the automatic pitch operation part and the additional pitch operation part, the surgical operator may intuitively perform the pitch operation, there is no need for a surgical assistant who has to adjust the position and the posture of the uterus, and the safety and the stability of the surgery may be obtained by directly adjusting the position and the posture of the uterus by the surgical operator.

### [DESCRIPTION OF THE DRAWINGS]

FIG. 1 is a schematic conceptual exploded view of a uterine manipulator according to an embodiment of the present disclosure.
FIGS. 2 to 4 are schematic conceptual views illustrating a portion (a pitch operation part) of an operation structure of a uterine manipulator according to an embodiment of the present disclosure.
FIG. 5A(a) is a schematic side view of a handle part in an unlocked state of an operation structure of a uterine manipulator according to an embodiment of the present disclosure, and FIG. 5A(b) is a schematic bottom view of the handle part in the unlocked state of the operation structure of the uterine manipulator, when viewed upward from a lower side, according to an embodiment of the present disclosure.
FIG. 5B(a) is a schematic side view of a handle part in a locked state of an operation structure of a uterine manipulator according to an embodiment of the present disclosure, and FIG. 5B(b) is a schematic bottom view of the handle part in the locked state of the operation structure of the uterine manipulator, when viewed upward from a lower side, according to an embodiment of the present disclosure.
FIGS. 6 to 8 are schematic conceptual views illustrating a portion (a yaw operation part) of an operation structure of a uterine manipulator according to an embodiment of the present disclosure.
FIG. 9 is a schematic cross-sectional view of a uterine manipulator according to an embodiment of the present disclosure coupled to a driving part of a uterine manipulator robot according to an embodiment of the present disclosure.
FIGS. 10A and 10B are schematic perspective views of a driving part of a uterine manipulator robot according to an embodiment of the present disclosure.
FIG. 11A is a schematic enlarged cross-sectional view of a portion of a uterine manipulator according to an embodiment of the present disclosure coupled to a driving part of a uterine manipulator robot according to an embodiment of the present disclosure.
FIG. 11B is a schematic enlarged cross-sectional view of a portion of a uterine manipulator according to an embodiment of the present disclosure.
FIG. 11C is an enlarged view of a portion of FIG. 11A.
FIG. 12 is a schematic enlarged cross-sectional view of a portion of a uterine manipulator according to an embodiment of the present disclosure coupled to a driving part of a uterine manipulator robot according to an embodiment of the present disclosure for describing driving of an automatic pitch manipulation part of a uterine manipulator robot according to an embodiment of the present disclosure.
FIG. 13 is a schematic enlarged cross-sectional view of a portion of a uterine manipulator according to an embodiment of the present disclosure coupled to a driving part of a uterine manipulator robot according to an embodiment of the present disclosure for describing driving of a yaw operation part of the uterine manipulator robot according to an embodiment of the present disclosure.
FIG. 14 is a schematic conceptual view of a grip part of a uterine manipulator robot according to an embodiment of the present disclosure.
FIG. 15 is a schematic conceptual enlarged perspective view of a portion of a uterine manipulator according to an embodiment of the present disclosure coupled to a driving part of a uterine manipulator robot according to an embodiment of the present disclosure for describing a grip part of the uterine manipulator robot according to an embodiment of the present disclosure.
FIG. 16 is a schematic conceptual perspective view of a portion of an automatic needle insertion part of a uterine manipulator robot according to an embodiment of the present disclosure.
FIG. 17 is a schematic conceptual side view of a portion of an automatic needle insertion part of a uterine manipulator robot according to an embodiment of the present disclosure.
FIGS. 18 and 19 are flowcharts illustrating operations of an automatic needle insertion part of a uterine manipulator robot according to an embodiment of the present disclosure.
FIGS. 20 to 24 are flowcharts for describing fastening to an end member of an operation module of a uterine manipulator robot according to an embodiment of the present disclosure.
FIG. 25 is a left side view of a uterine manipulator according to an embodiment of the present disclosure.
FIG. 26 is a left side view and a plan view of a uterine manipulator according to an embodiment of the present disclosure, in which illustration of an additional connection member is omitted.
FIG. 27 is a schematic conceptual perspective view of a uterine manipulator system according to an embodiment of the present disclosure.
FIG. 28 is a schematic conceptual operation view of a uterine manipulator system according to an embodiment of the present disclosure for describing an additional pitch operation part.
FIG. 29 is a schematic operation view of a uterine manipulator system according to an embodiment of the present disclosure for describing a roll operation part.
FIG. 30 is a conceptual perspective view of a first rail structure and a second rail structure of a uterine manipulator system according to an embodiment of the present disclosure.

### [BEST MODE]

Hereinafter, embodiments of the present disclosure will be described in detail with reference to the accompanying drawings such that those skilled in the art may easily implement the present disclosure. However, the present disclosure may be implemented in various different forms and is not limited to the embodiments described herein. Moreover, in the drawings, portions irrelevant to the description are omitted to clearly describe the present disclosure, and similar reference numerals is assigned to similar portions throughout the specification.

Throughout the specification, when it is supposed that a portion is "connected" to another portion, this includes not only "directly connected" but also "electrically connected" to other elements in between.

Throughout the specification, when it is supposed that a member is located on another member "on", "in an upper portion", "at an upper end", "under", "in a lower portion", "at a lower end", this includes not only the case where one member is in contact with another member but also the case where another member is present between two other members.

Throughout the specification, when a portion "comprises" a component, it means that it can further include other component, without excluding other components unless specifically stated otherwise.

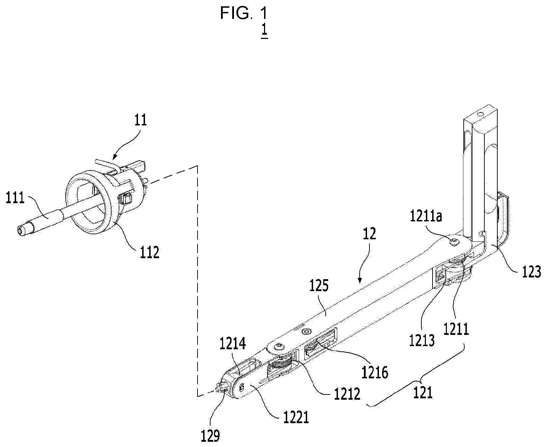

Terms (an upper side, an upper surface, a lower side, a lower surface, a left side, a right side, a front side, a front end, a rear side, a rear end, and the like) related to directions or positions in the description of the embodiment of the present disclosure are set based on a disposition state of the components illustrated in the drawing. For example, referring to FIG. 1, a direction substantially of 12 o'clock may be an upper side, a surface that substantially faces the direction of 12 o'clock may be an upper surface, a surface that substantially faces 6 o'clock may be a lower surface, a direction substantially of 4 o'clock may be a left side, a direction substantially of 10 o'clock may be a right side, a direction substantially of 8 o'clock may be a front side, an end that substantially faces the direction of 8 o'clock may be a front end, a direction substantially of 2 o'clock may be a rear side, and an end that substantially faces the direction of 2 o'clock may be a rear end.

The present disclosure relates to a uterine manipulator and a uterine manipulator robot system.

First, a uterine manipulator (hereinafter, referred to as "the manipulator") 1 according to an embodiment of the present disclosure will be described.

Referring to FIG. 1, the manipulator 1 includes an operation module 11. The operation module 11 includes an end tip 111. The end tip 111 may be fixed in an interior of a uterus to control a position and a posture of the uterus. Furthermore, the operation module 11 may include a cup 112 that is provided to surround a rear end of the end tip 111. Furthermore, the operation module 11 may include a needle 246 that is provided inside the cup 112. The needle 246 may be used to pass through a cervix and inject a drug. The needle 246 will be described later.

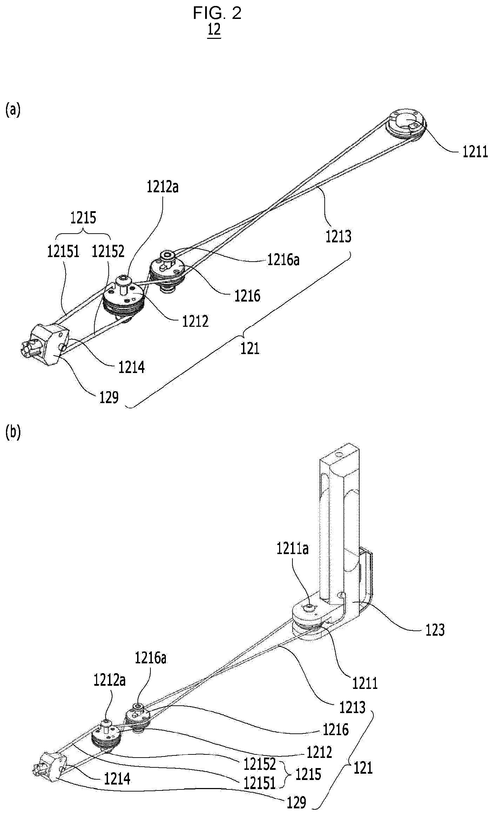

Furthermore, referring to FIGS. 1 and 2, the present manipulator 1 includes an operation structure 12. Referring to FIGS. 1 to 4, the operation structure 12 includes an end member 129 that is provided with an operation module 11 at a front end thereof. The operation module 11 may be mounted on a front end of the end member 129 to be attached and detached (mounted and demounted).

Furthermore, referring to FIGS. 2 to 4, the operation structure 12 includes a pitch operation part 121 that enables a pitch operation of rotating the end member 129 about a first axis such that a pitch in a direction which the end tip 111 faces is adjusted. For example, the first axis may mean a leftward/rightward axis. That is, the pitch operation may mean an operation that is achieved by rotating the end member 129 while the leftward/rightward direction is taken as a rotation axis thereof. Accordingly, an angle in an upward/downward direction with respect to a horizontal direction to a direction which the end tip 111 faces may be adjusted.

Specifically, referring to FIGS. 2 to 4, the pitch operation part 121 includes a first joint 1211 that is disposed to be rotatable while an upward/downward direction is taken as a rotation axis thereof. The first joint 1211 may be formed in a shape of a disk. A rotary shaft (a first rotary shaft 1211a) may cross a center of the first joint 1211 in the upward/downward direction.

Furthermore, referring to FIGS. 2 to 4, the pitch operation part 121 includes a second joint 1212 that is disposed on a front side of the first joint 1211. The second joint 1212 is disposed to be rotatable while the upward/downward direction is taken as a rotation axis thereof. The second joint 1212 may be formed in a shape of a disk. Furthermore, a rotary shaft (a second rotary shaft) may cross a center of the second joint 1212 in the upward/downward direction.

Furthermore, referring to FIGS. 2 to 4, the pitch operation part 121 includes a first connection part 1213. The first connection part 1213 may connect the first joint 1211 and the second joint 1212. For example, when the first joint 1211 is rotated, the first connection part 1213 transmits a rotational force of the first joint 1211 to the second joint 1212 to rotate the second joint 1212. The first connection part 1213 may be rigid. Alternatively, preferably, it may be a configuration that may maintain a stretched state of the first connection part 1213, for example, the first connection part 1213 may be a wire. When the first connection part 1213 is rigid, the first connection part 1213 may be deformed in a process of transmitting the rotational force. In consideration of this, the first connection part 1213 preferably may be a wire.

Furthermore, the first joint 1211 and the second joint 1212 may have a shape of a disk as described above, and a groove, in which a portion that surrounds the first joint 1211 and the second joint 1212 of the wire is accommodated, may be recessed from the outer surface to a radially inner side of each of the first joint 1211 and the second joint 1212 to extend in the circumferential direction to prevent the first connection part (a wire) 1213 that surrounds the first joint 1211 and the second joint 1212 from being separated from the first joint 1211 and the second joint 1212.

For example, the first connection part 1213 may be two rows of wire that are provided to extend forward while surrounding at least a portion of the first joint 1211 and surround at least a portion of the second joint 1212. In this case, the first connection part 1213 may be configured such that the second joint 1212 is rotates in the same direction when the first joint 1211 is rotated.

Furthermore, in this case, referring to FIGS. 2 to 4, the pitch operation part 121 may include a sixth joint 1216 that is provided between the first joint 1211 and the second joint 1212 to guide the operation of the wire while supporting the wire. When a distance between the first joint 1211 and the second joint 1212 is long, it may be difficult for the rotational force of the first joint 1211 to be completely transmitted to the second joint 1212. In consideration of this, the sixth joint 1216 may be provided between the first joint 1211 and the second joint 1212 so that the rotational force of the first joint 1211 may be transmitted to the second joint 1212 as much as possible. Furthermore, in this case, the first connection part 1213 provided with two rows of wires may extend between the first joint 1211 and the sixth joint 1216 to be misaligned with each other in the form of "X".

Furthermore, the sixth joint 1216 may have a shape of a disk. In addition, in the sixth joint 1216, a groove, in which a portion of the wire, which contacts the sixth joint 1216, is accommodated, may be recessed from the outer surface to a radially inner side of the sixth joint 1216 to extend in a circumferential direction thereof such that the first connection part (a wire) 1213 that extends while contacting it may be stably supported by the sixth joint 1216. Accordingly, the wire may not be separated from the sixth joint 1216 and may be stably guided by the sixth joint 1216.

Furthermore, referring to FIGS. 2 to 4, the pitch operation part 121 includes a third joint 1214. The third joint 1214 is disposed on a front side of the second joint 1212. The third joint 1214 is provided with an end member 129 at a front end thereof. Furthermore, the third joint 1214 is disposed to be rotatable while the leftward/rightward direction is taken as a rotation axis thereof. As the third joint 1214 is rotated while the leftward/rightward direction as a rotation axis thereof, the end member 129 may be rotated while the leftward/rightward direction as a rotation axis thereof, and accordingly, the pitch operation may be implemented.

Furthermore, referring to FIGS. 2 to 4, the pitch operation part 121 includes a second connection part 1215 that rotates the third joint 1214 by transmitting the rotational force of the second joint 1212 to the third joint 1214 when the second joint 1212 is rotated.

Referring to FIGS. 2 to 4, the second connection part 1215 may include a left connection member 12152 that extends forward from a left portion of the second joint 1212 and is connected to a left portion of the third joint 1214, and a right connection member 12151 that extends forward from a right portion of the second joint 1212 and is connected to a right portion of the third joint 1214. Because the third joint 1214 is configured to be rotated while the leftward/rightward direction is taken as a rotation axis thereof, the left connection member 12152 may be pulled and rotated in one direction when the second joint 1212 is rotated in one direction. When the second joint 1212 is rotated in an opposite direction, the right connection member 12151 may be pulled and rotated in an opposite direction. In this way, because the right connection member 12151 is rotated in one direction or an opposite direction while the left and right direction of the third joint 1214 is taken as an axis thereof, a pitch operation may be performed. For reference, the one direction of the second joint 1212 and the one direction of the third joint 1214 may be the same or different from each other. Furthermore, the other direction of the second joint 1212 and the other direction of the third joint 1214 may be the same or different from each other.

Furthermore, referring to FIG. 2, one of the left connection member 12152 and the right connection member 12151 may extend from an upper portion of the second joint 1212 to be connected to an upper portion of the third joint 1214, and the other one may extend from a lower portion of the second joint 1212 to be connected to a lower portion of the third joint 1214. The third joint 1214 may have a state, in which it stands up such that a rotation axis thereof extends in the leftward/rightward direction and the second joint 1212 is in a laid state, in which it is perpendicular to the third joint 1214 such that a rotation axis thereof extends in the upward/downward direction, thus, as the second joint 1212 is rotated, heights of front ends of the left connection member 12152 and right connection member 12151 connected to the third joint 1214 in the upright state may be changed, and they may be distorted (or misaligned or twisted) without being parallel to each other.

To cover twisting, and further interferences caused by the rotation of the third joint 1214 in this upright state, a rear end of any one of the left connection member 12152 and the right connection member 12151 is connected to a lower height position of the second joint 1212, and a rear end of the other one is connected to an upper height position (a position that is higher than the lower height position) of the second joint 1212 so that a rotation angle range may be set within a range, in which twisting does not occur. That is, the rear ends of the plurality of connection members 12151 and 12152 are connected to the second joint 1212 at different heights, and the rotation angle range is set such that the connection members 12151 and 12152 are not twisted, and thus, as the rotation of the first joint 1211 is transmitted forward while preventing (covering) twisting (mutual interferences) due to the rotation of the third joint 1214 in the upright state, it may finally be converted into pitch operation by the third joint 1214 in the upright state, and thus, the pitch operation of the end tip may be performed within a specific angle range. However, in consideration of the fact that the angle range of the pitch operation may be limited to prevent twisting (interferences), the manipulator 1 may be provided with an additional pitch operation part 26 in the driving part 2 that will be described later to give an additional degree of freedom to further expand the angle range of the pitch operation.

Furthermore, in the case of the connection members described in the present disclosure, particularly, the left connection member 12152 and the right connection member 12151, may be connected to the corresponding joints, respectively, in a stopping fastening form (a hinge fastening form), by which the front ends and the rear ends thereof may maintain the connection even when they are slightly rotated as in a ring fastening form so that the connection states may be maintained to a degree even when they are not parallel to each other and slightly misaligned from each other (even when they are twisted). For example, the front ends of the left connection member 12152 and the right connection member 12151 may be connected in a stopping fastening form (a hinge fastening form) to a specific eccentric position that is eccentric from the center of the third joint 1214 in an upright state, and this connection may be made by a connection method that may cover the occurrence of a specific rotation or displacement at the front ends due to the occurrence of twisting during rotation interworking between the third joint 1214 in an upright state and the second joint 1212 in a laid state, in a specific accommodation range.

As described above, when among the left connection member 12152 or the right connection member 12151, the connection member connected to the upper portion is moved rearward, the other connection member may be moved forward, and the third joint 1214 may be rotated in one direction so that a direction which the end tip faces may face an upper side. Furthermore, when among the left connection member 12152 and the right connection member 12151, the connection member connected to the upper portion is moved forward, the other connection member may be moved rearward, and the third joint 1214 may be rotated in an opposite direction so that a direction which the end tip faces may face to a lower side. Accordingly, the pitch of the end tip 11 may be adjusted.

That is, the pitch operation part 121 includes a first joint 1211 and a second joint 1212 that are disposed in the horizontal direction, and includes a third joint 1214 that is disposed in the vertical direction, and the rotation of the first joint 1211 allows the end member 129 to perform a pitch operation through the first connection part 1213 and the second connection part 1215.

Furthermore, referring to FIGS. 2 to 4, the operation structure 12 may include a handle part 123 that is connected to a rotary shaft 1211a (the above-described first rotary shaft 1211a) of the first joint 1211 to manipulate the rotation of the first joint 1211. The handle part 123 may be configured to interwork with the rotation of the first joint 1211. Furthermore, the handle part 123 and the first joint 1211 may be configured to be rotated about the first rotary shaft 1211a (that is, when the first joint 1211 and the handle part 123 are rotated, the first rotary shaft 1211a may not be rotated). Accordingly, when the handle part 123 is rotated, the first joint 1211 may be rotated to perform the above-described pitch operation. At this time, depending on the direction, in which the handle part 123 is rotated, the first joint 1211 may rotate in one direction or an opposite direction, and depending on the direction, in which the first joint 1211 is rotated, the end tip 111 may face an upper side or face to a lower side (for example, when the handle part 123 is rotated in one direction, the end tip 111 may face an upper side, and when the handle part 123 is rotated in an opposite e direction, the end tip 111 may face a lower side).

For reference, the first rotary shaft 1211a may be configured such that an upper portion thereof protrudes toward an upper side of the first joint 1211 or a lower portion thereof protrudes toward a lower side of the first joint 1211. In this case, as will be described later, the first rotary shaft 1211a may be formed as one with a locking shaft 1255 of a fifth joint 1223, and a portion of the first rotary shaft 1211a, which protrudes toward a lower side of the first joint 1211, may form the locking shaft 1255. In this case, the handle part 123 may include an upper portion and a lower portion (portions that surround the locking shaft 1255) of the first rotary shaft 1211a.

Furthermore, referring to FIG. 1, the present manipulator 1 may include a case 125 including an upper surface that extends from an upper side of the first joint 1211 to an upper side of the second joint 1212, a lower surface that extends from a lower side of the first joint 1211 to a lower side of the second joint 1212, a left surface that extends from a left side of the first joint 1211 to a left side of the second joint 1212 and a right surface that extends from a right side of the first joint 1211 to a right side of the second joint 1212. For reference, a portion of the case 125 may be opened such that an inside and an outside thereof communicate with each other. Furthermore, a rotary shaft (the second rotary shaft) of the second joint may be fastened to the case 125 to be rotatable, a sixth rotary shaft (the sixth rotary shaft) of the sixth joint may be fastened to the case 125 to be rotatable, and a rotary shaft (the first rotary shaft 1211a) of the first joint 1211 may be fastened to the case to be rotatable.

In this case, the operator may hold the case 125 and manipulate the handle part 123 so that the first rotary shaft 1211a is rotated with respect to the case 125.

As described above, the handle part 123 may serve as a manual manipulation part. That is, the handle part 123 may be configured to enable a manual operation of the manipulator 1, and the driving part 2 that will be described later may be configured to automate the driving of the manipulator 1 by using a motor.

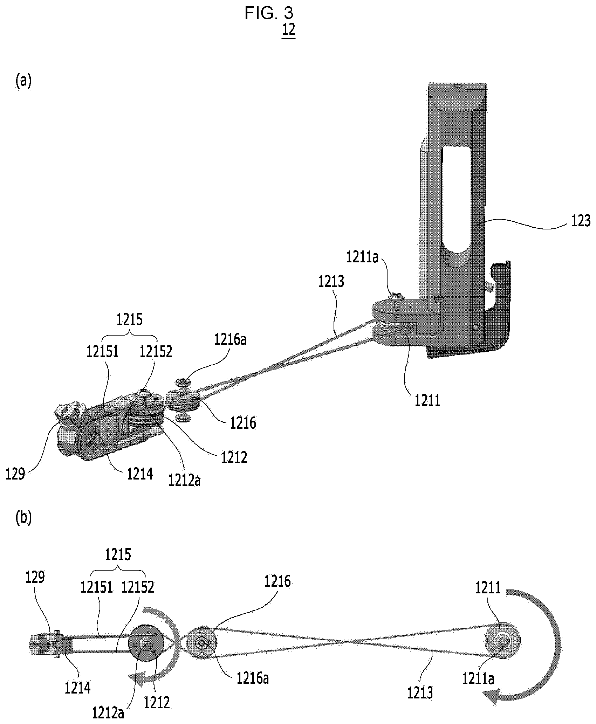

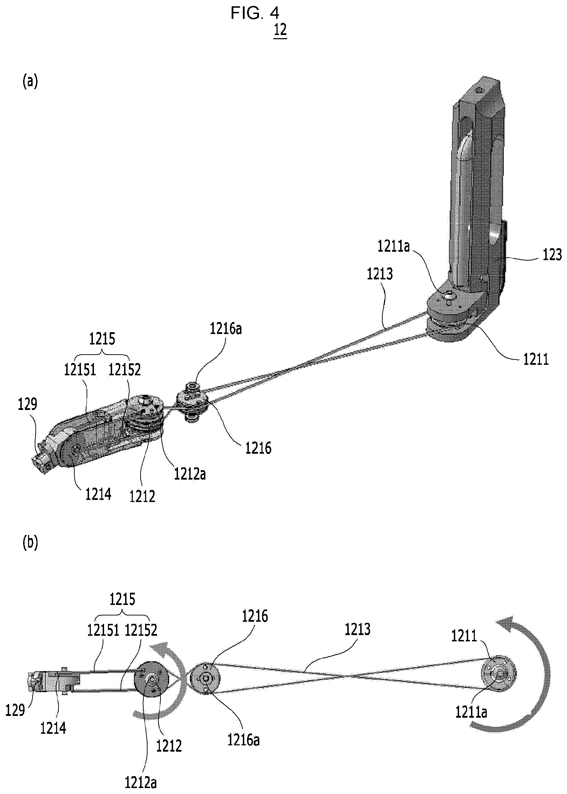

In comparison of FIGS. 2 and 3, the user may rotate the handle part 123 in one direction with respect to the case 125, and in this case, the first joint 1211 is rotated in one direction, and the second joint 1212 is rotated so that the front end of the end member 129 faces an upper side and the angle from the horizontal direction may be increased, and in comparison of FIGS. 2 and 4, the user may rotate the handle part 123 in an opposite direction with respect to the case 125, and in this case, the first joint 1211 is rotated in an opposite direction, and the second joint 1212 is rotated so that the direction which the front end of the end member 129 faces may face a lower side and the angle from the horizontal direction may be increased.

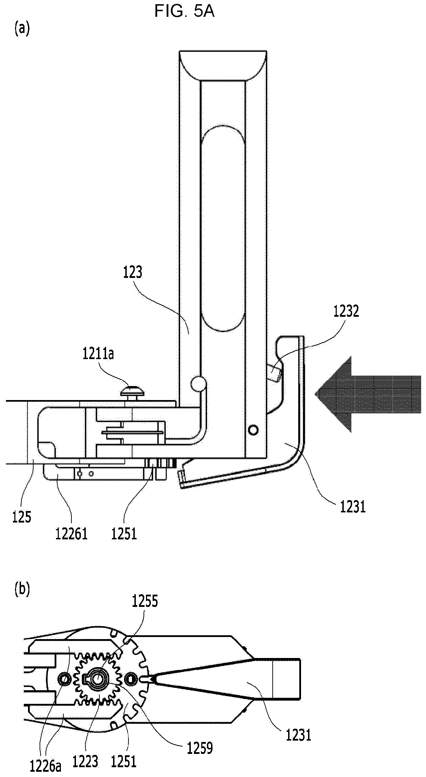

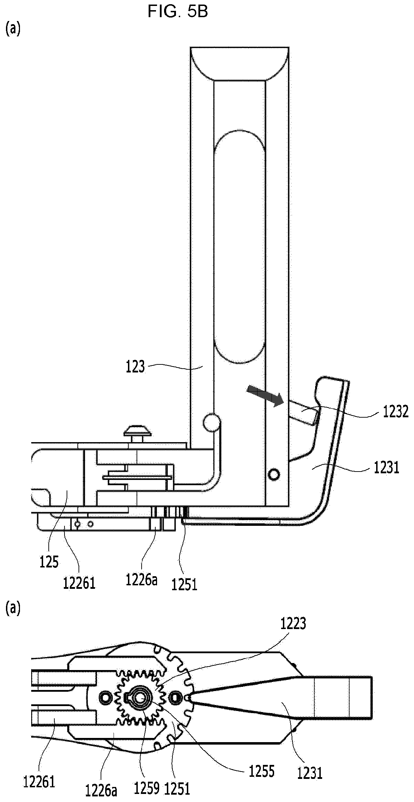

Furthermore, referring to FIGS. 5A and 5B, the handle part 123 may be locked not to be rotated with respect to the case 125, or may be unlocked to be rotated. This may be done by the ratchet principle.

For example, referring to FIGS. 5A and 5B, a fixing tooth 1251 having a sawtooth may be provided at least a rear end of the case 125, for example, of a lower surface of the case 125, and an engagement part 1231 that may be selectively engaged with the fixing tooth 1251 may be provided in the handle part 123. The engagement part 1231 may include a first portion that extends in the upward/downward direction, and a second portion that extends forward from a lower end of the first portion to be engaged with the fixing tooth 1251, and the engagement part 1231 may be hinge-connected to the handle part 123, so that a front end of the second portion is engaged with the tooth 1251, or a front end of the second portion is selectively disengaged from the tooth 1251. Referring to FIG. 5A, when the second portion is not engaged with the tooth 1251, the handle part 123 may be rotatable with respect to the case 125. Furthermore, referring to FIG. 5B, when the second portion is engaged with the tooth 1251, the handle part 123 may be prevented from being rotated with respect to the case 125 (a rotation locked state). Furthermore, referring to FIGS. 5A and 5B, the handle part 123 may be provided with a lever 1232 that may selectively enable an engagement state and a disengagement state with respect to the tooth 1251 of the engagement part 1231. When the rear end of the handle part 1231 is rotated forward and the handle part 1231 is operated while the engagement part 1231 and fixing tooth 1251 are engaged from each other, the engagement part 1231 and the teeth 1251 be disengaged, and when the rear end of the handle part 123 is rotated rearward in the disengaged state, the engagement part 1231 and the teeth 1251 may be engaged with each other, and the lever 1232 may guide an operation related to the engagement of the handle part 1231.

According to a conventional manipulator, an angle of the manipulator cannot be fixed so that it is not easy to use it because another operation cannot be performed after the operator fixes the pitch angle even when it is desired. On the other hand, according to the manipulator 1, when the operator wants to fix the pitch state of the manipulator, the operator engages a tip of the engagement part 1231 of the handle part 123 with the teeth of the fixing tooth 1251, and thus, the rotation of the first joint 1211 may be prevented to fix the pitch state by preventing the rotation of the handle part 123, and the handle part 123 and the first joint 1211 may be rotatable so that the pitch state may be adjusted, as the operator disengages the tip of the engagement part 1231 of the handle part 123 from the tooth of the fixing tooth 1251.

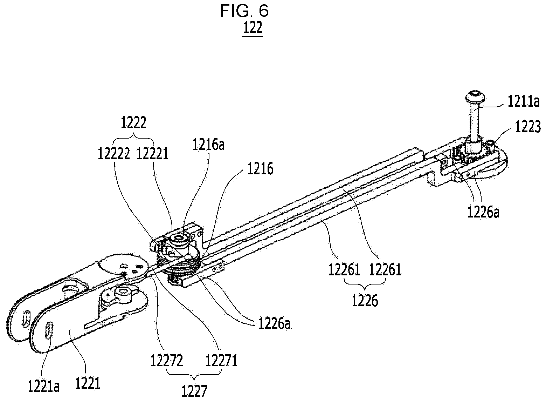

Furthermore, referring to FIG. 6, the operation structure 12 includes a yaw operation part 122. The yaw operation part 122 enables a yaw operation centered on the second axis of the pitch operation part 121 so that a yaw in a direction which the end tip 111 faces is adjusted. For example, the second axis may mean an upward/downward direction axis. That is, the yaw operation may mean an operation that is performed by rotating the end member 129 while the upward/downward direction is taken as a rotation axis thereof. Accordingly, angles in leftward/rightward direction with respect to the forward/rearward direction which the end tip 111 faces may be adjusted.

Referring to FIG. 6, the yaw operation part 122 may include a mounting part 1221, on which the third joint 1214 is mounted.

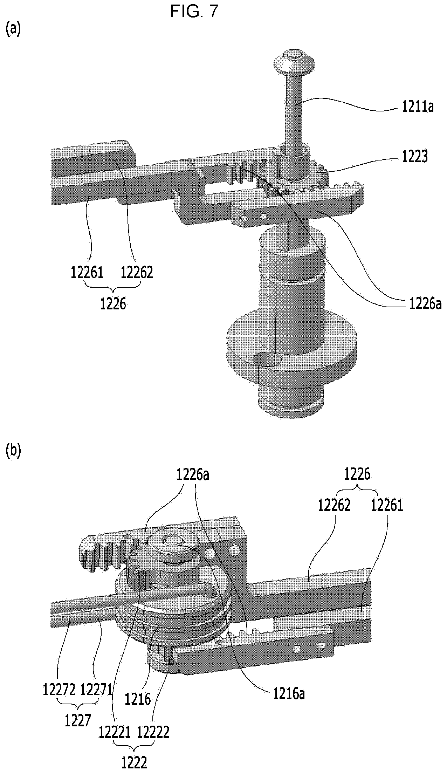

Furthermore, referring to FIGS. 6 and 7B, the yaw operation part 122 may include a fourth joint 1222 that is disposed on a rear side of the mounting part 1221 and is disposed to be rotatable while the upward/downward direction is taken as a rotation axis thereof.

Furthermore, referring to FIGS. 6 and 7A, the yaw operation part 122 may include a fifth joint 1223 that is disposed on a rear side of the fourth joint 1222 and disposed to be rotatable while the upward/downward direction is taken as a rotation axis thereof. The fifth joint 1223 may be formed in a shape of a disk. Furthermore, a locking shaft 1255 may be disposed in the fifth joint 1223 to cross a center of the fifth joint 1223 in the upward/downward direction.

Furthermore, referring to FIG. 6, the yaw operation part 122 may include a first link part 1226 that transmits a rotational force of the fifth joint 1223 to the fourth joint 1222 when the fifth joint 1223 is rotated, or transmits a rotational force of the fourth joint 1222 to the fifth joint 1223 when the fourth joint 1222 is rotated.

Furthermore, referring to FIGS. 6 and 7A, the fifth joint 1223 may be a pinion gear. Furthermore, the first link part 1226 may include two rows of first link members 12261 and 12262 that extend forward. Each of the first link members 12261 and 12262 may be provided with a rack gear 1226a that is engaged with each of a left portion and a right portion of the fifth joint 1223 at a rear end thereof.

Furthermore, a rack gear 1226a may be provided at a front end of each of the first link members 12261 and 12262 in the two rows.

Furthermore, the fourth joint 1222 may include a left pinon gear 12222 that is engaged with a rack gear 1226a of a front end of the left one of the first link members 12261 and 12262 in the two rows, and a right pinion gear 1221 that is engaged with a rack gear 1226a of a front end of the right one of the first link members 12261 in the two rows.

Furthermore, referring to FIG. 6, the yaw operation part 122 may include a second link part 1227 that rotates the mounting part 1221 in the rotational direction of the fourth joint 1222 by transmitting a rotational force of the fourth joint 1222 to the mounting part 1221 when the fourth joint 1222 is rotated.

Furthermore, as described above, the pitch operation part 121 may include a sixth joint 1216 that is provided between the first joint 1211 and the second joint 1212 to guide an operation of the wire while supporting the wire, and one of a left pinion gear 12222 and a right pinion gear 12221 may be provided at one of an upper portion and a lower portion of a rotary shaft (the sixth rotary shaft) 1216a of the sixth joint 1216. Furthermore, the other one of the left pinion gear 12222 and the right pinion gear 12221 may be provided at the other one of the upper portion and the lower portion of the rotary shaft (the sixth rotary shaft) 1216a of the sixth joint 1216.

Accordingly, the rotation of the fourth joint 1222 may mean rotation of the sixth joint 1216, and the sixth joint 1216 may be rotated when a rotational force is applied to the fourth joint 1222 by the first link part 1226.

Furthermore, the second link part 1227 may include a second upper link member 12271 that extends from an upper portion of the sixth joint 1216 and is connected to an upper portion of the mounting part 1221, and a second lower link member 12272 that extends from a lower portion of the sixth joint 1216 and is connected to a lower portion of the mounting part 1221.

As described, when the sixth joint 1216 is rotated, the mounting part 1221 may be rotated in a direction, in which the sixth joint 1216 is rotated, while interworking with the rotation of the sixth joint 1216, and thus, the yaw operation of the operation module 11 may be performed.

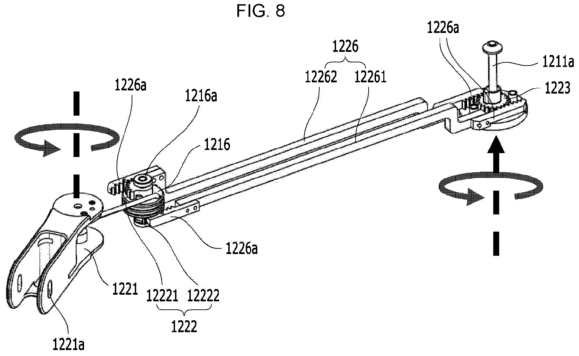

Furthermore, referring to FIG. 8, when the fifth joint 1223 is rotated, the sixth joint 1216 may be rotated in the rotation direction, and the mounting part 1221 may be rotated in a direction, in which the sixth joint 1216 is rotated, while interworking with the rotation of the sixth joint 1216, and thus, the yaw operation may be performed while the operation module 11 is rotated in the rotational direction. Accordingly, when the operation module 11 needs to be rotated in an opposite direction, the operation module 11 may be rotated in the opposite direction when the fifth joint 1223 is rotated in an opposite direction.

Compared to the existing technology, the manipulator 1 may implement a light weight by totally changing a mechanism (a structure or configurations and a connection/interworking relationship thereof), and a manipulation method may be achieved in an intuitive manner, so that the operator may manipulate proficiently.

Furthermore, for example, the manual manipulation part of the manipulator 1 may be used in the same manner as an existing surgical method (used in the same manner as an existing **RUMI** manipulation) before being coupled to a driving part 2 that will be described later.

Hereinafter, a uterine manipulator robot (hereinafter, referred to as "the robot") according to an embodiment of the present disclosure will be described. However, the robot includes the manipulator 1 described above and shares the same or corresponding technical features and configurations to those of the manipulator 1. Accordingly, a repeated description of the description described in the manipulator 1 will be simplified or omitted, and the same reference numerals will be used for the same or similar configurations.

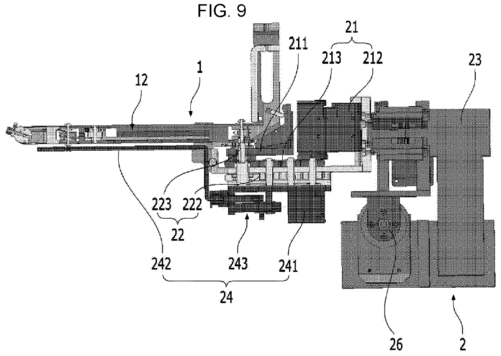

Referring to FIG. 9, the robot includes the manipulator 1 described above.

The manipulator 1 includes a handle part 123 that is connected to the rotary shaft of the first joint 1211 to manipulate the rotation of the first joint 1211.

Furthermore, referring to FIG. 9, the robot includes the driving part 2 that drives the operation structure 12. The handle part 123 may be provided to enable manual operation of the manipulator 1, and the driving part 2 described later may be provided to automate the driving of the manipulator 1 using a motor. The manipulator 1 enables rotation of two degrees of freedom through an association with the driving part 2, and for example, enables a yaw operation and a pitch operation, but as will be described later, three degrees of rotation, such as a yaw operation, a pitch operation, and a roll operation, is enabled in consideration of an association with a roll operation part 23.

According to the present disclosure, convenience of user may be improved (coupled to be used during an automatic manipulation and only the manual manipulator part is used when a manual manipulator is necessary) by separating the handle part 123 that serves as the manual manipulation part and the driving part 2 that serves as an automation manipulation part.

Referring to FIGS. 9 to 10B, the driving part 2 includes a seating part 211, in which a rear end of the manipulator 1 is seated. The manipulator 1 is detachable from the seating part 211.

Furthermore, referring to FIGS. 9 to 10B, the driving part 2 includes an automatic pitch manipulation part 21 that rotates the handle part 123 seated on the seating part 211.

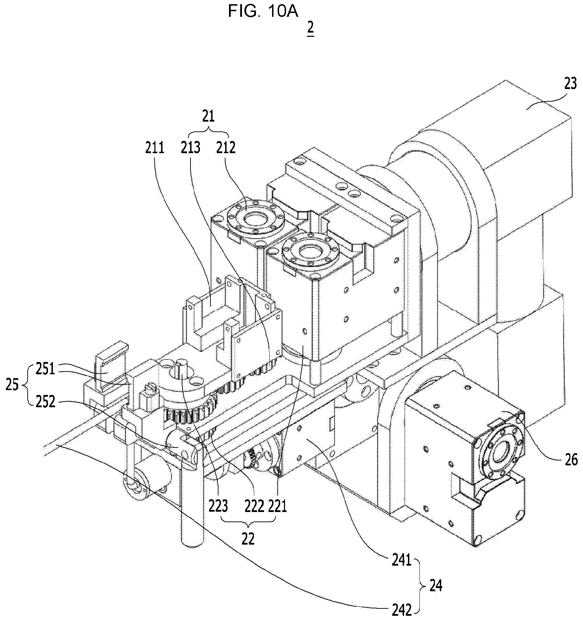

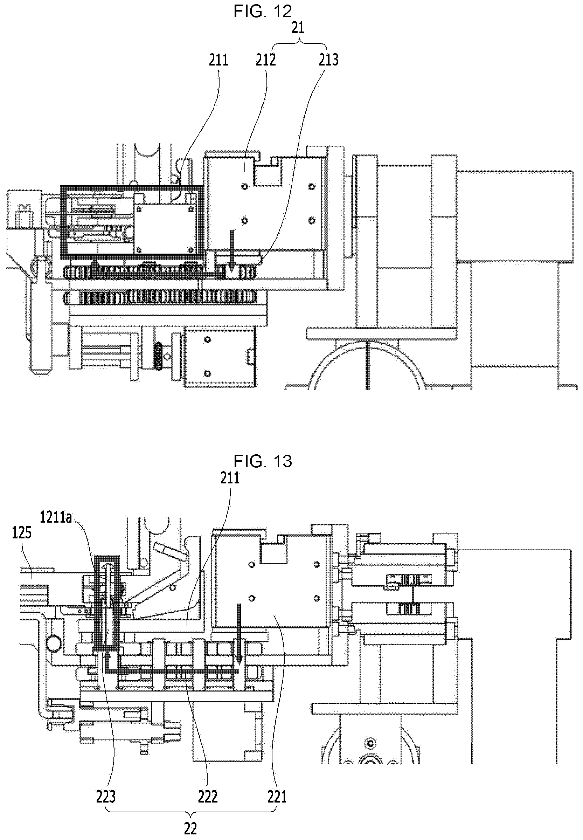

Referring to FIGS. 10A to 12, the automatic pitch manipulation part 21 may include a motor 212, and a gear structure 213 that transmits a rotational force of the motor 212 to the handle part 123. The gear structure 213 may include a first gear that is provided to surround a rotary shaft of the motor 212 to be rotated through the rotation of the rotary shaft, a second gear that is provided on a lower side of the seating part 211 such that the seating part 211 is rotated during rotation thereof, and a third gear that transmits the rotation of the first gear to the second gear. One or more third gears may be provided. When a rotational force of the motor 212 is transmitted to the second gear through the first gear and the third gear, and the second gear is rotated, the seating part 211 may be rotated.

Furthermore, the manipulator 1 includes a yaw operation part 122. The yaw operation part 122 enables a yaw operation centered on the second axis of the end member 129 so that a yaw in a direction which the end tip faces is adjusted.

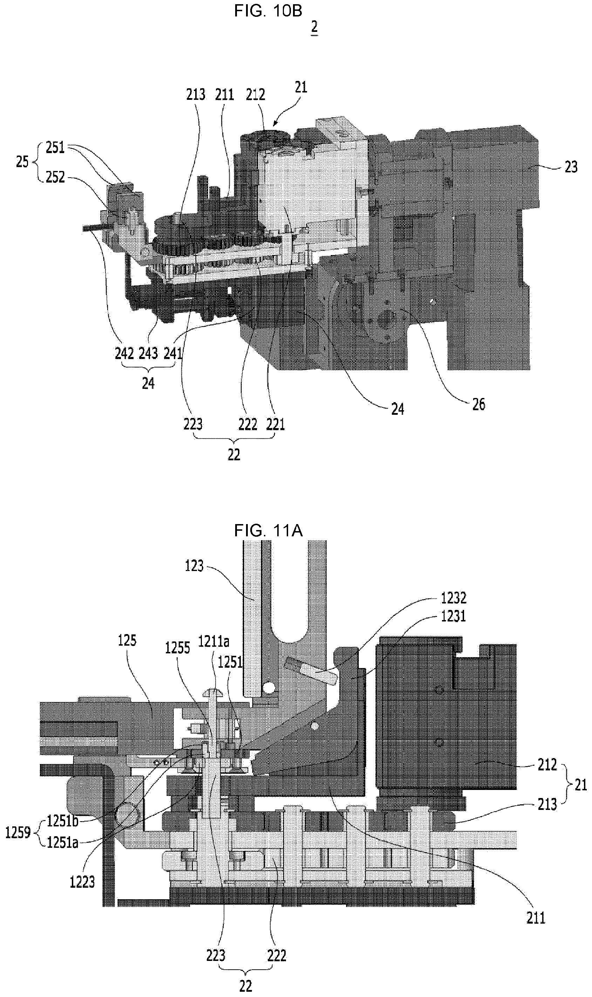

Furthermore, referring to FIGS. 10B, 11A, and 13, the robot 2 includes a yaw operation manipulating part 22 that operates the yaw operation part 122.

Referring to FIGS. 10B, 11A, and 13, the yaw operation part 122 may include a mounting part 1221, on which the third joint 1214 is mounted.

Furthermore, the yaw operation part 122 may include a fourth joint 1222 that is disposed on a rear side of the mounting part 1221 and is disposed to be rotatable while the upward/downward direction is taken as a rotation axis thereof.

Furthermore, the yaw operation part 122 may include a fifth joint 1223 that is disposed on a rear side of the fourth joint 1222 and disposed to be rotatable while the upward/downward direction is taken as a rotation axis thereof. Furthermore, referring to FIG. 11A, the yaw operation part 122 may include a locking shaft 1255 that crosses the fifth joint 1223 vertically.

In other words, as described above, the fifth joint 1223 may be formed in a shape of a disk. Furthermore, a locking shaft 1255 may be disposed in the fifth joint 1223 to cross a center of the fifth joint 1223 in the upward/downward direction.

Referring to FIG. 11A, the fifth joint 1223 may be provided on a lower side of the first joint 1211. Furthermore, the rotary shaft 1211a of the first joint 1211 and the locking shaft 1255 of the fifth joint 1223 may be connected to each other vertically.

Furthermore, the yaw operation part 122 may include a first link part 1226 that transmits a rotational force of the fifth joint 1223 to the fourth joint 1222 when the fifth joint 1223 is rotated, or transmits a rotational force of the fourth joint 1222 to the fifth joint 1223 when the fourth joint 1222 is rotated.

Furthermore, the yaw operation part 122 may include a second link part 1227 that rotates the mounting part 1221 in the rotational direction of the fourth joint 1222 by transmitting a rotational force of the fourth joint 1222 to the mounting part 1221 when the fourth joint 1222 is rotated.

Furthermore, referring to FIGS. 10B, 11A, and 13, the yaw operation manipulating part 22 may include a spline 223 that is configured to protrude upward from the seating surface of the seating part 211 to move the locking shaft 1255 of the fifth joint 1223 upward when the manipulator 1 is seated on the seating part 211, to demount the locking shaft 1255 of the fifth joint 1223 from the fifth joint 1223, and to replace the locking shaft 1255 of the fifth joint 1223.

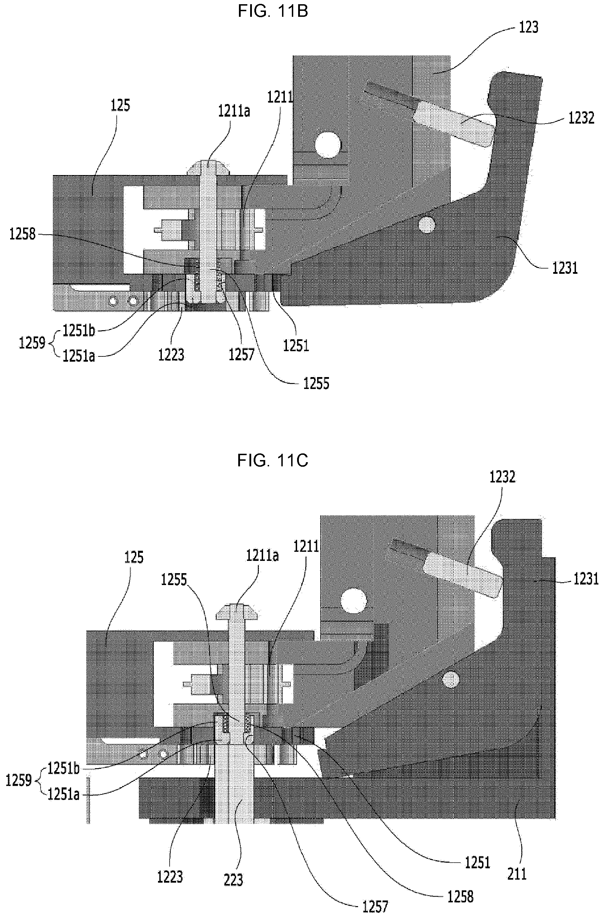

For example, referring to FIG. 11B, the locking shaft 1255 of the fifth joint 1223 may extend downward from the first rotary shaft 1211a that is the rotary shaft of the first joint 1211 to be inserted upward into the hole of the fifth joint 1223, and the locking shaft 1255 of the fifth joint 1223 may pass holes 1257 that are formed at a portion located on a lower side of the first joint 1211 of the handle part 123 and a portion located on a lower side of the first joint 1211 of a lower surface of the case 125 from the first rotary shaft 1211a, and may be inserted upward into the hole formed in the fifth joint 1224. In this case, referring to FIG. 11B, a fixing unit 1259 including a support member 1251a that extends radially outward from the locking shaft 1255 may be provided at a lower end of the locking shaft 1255, and the portion, through which the locking shaft 1255 passes, may be formed at a lower portion of the portion located on a lower side of the first joint 1211 and on a lower surface of the case 125 to be expanded radially outward such that the fixing unit 1259 is accommodated, and thus, the support member 1251a may face a portion that surrounds the locking shaft 1255 at the upper portion of the portion located on the lower side of the first joint 1211 of the handle part 123, and an elastic member 1258 may be interposed in a space between a surface that faces the support member 1251a of the handle part 123 and the support member 1251a. Furthermore, referring to FIG. 11B, the fixing unit 1259 may include a circumferential member 1251b that protrudes to an upper side of the support member 1251a at a radially outer end of the support member 1251a, and a lower portion of the elastic member may be located between the circumferential member 1251b and the locking shaft 1255. Accordingly, the elastic member may be stably located. Furthermore, the elastic member may be a spring that is provided to surround the locking shaft 1255.

As described above, referring to FIG. **11B****,** in a state, in which the manipulator 1 is not seated on the seating part 211, the elastic member is expanded (an elastic restoration state) so that at least portions (lower portions of the locking shaft 1255 and the fixing unit 1259) of the locking shaft 1255 and the fixing unit 1259 may be disposed in the hole of the fifth joint 1223, at least portions (for example, upper portions of the locking shaft 1255 and the fixing unit 1259) of the locking shaft 1255 and the circumferential member 1251b may be located in a hole 1257 of the case 125, in this case, referring to FIG. 5A(b) and 5B(b), a horizontal cross-sectional shape of a radially outer periphery of the fixing unit 1259 may be a shape having a circular portion and a protrusion that protrudes radially outward from the circular portion, the hole that surrounds the locking shaft 1255 and the fixing unit 1259 of the fifth joint 1223 and the hole that surrounds the locking shaft 1255 and the fixing unit 1259 of the fifth joint 1223 of the case 125 may have a shape that is engaged with a horizontal cross-sectional shape of the radially outer periphery of the fixing unit 1259, and thus, the fifth joint 1223 is not rotated due to engagement so that a yaw operation is not performed in the state, in which the manipulator 1 is not seated on the seating part 211.

Furthermore, referring to FIGS. 11C, when the manipulator 1 is seated on the seating part 211, the spline 223 may push the locking shaft 1255 and the support member 1251a upward and compress the elastic member 1258 so that the locking shaft 1255 and the fixing unit 1259 are located in the original positions (the elastic member 150 may be compressed), and thus, the rotation locking state of the fifth joint 1223 may be released and the fifth joint 1223 may be rotated by the spline 223. For reference, it is preferable that the spline 223 has a diameter that exceeds at least an outer diameter of the locking shaft 1255 to cover the locking shaft 1255 and the fixing unit 1259, and it is more preferable that it has a diameter of the outer diameter of the fixing unit 1259 or more.

Furthermore, when the manipulator 1 is demounted from the seating part 211, the elastic member 1258 may be elastically restored, and the locking shaft 1255 and the fixing unit 1259 may be restored to be located in the hole of the fifth joint 1223, and the yaw operation may be locked.

The manipulator 1 may be mounted in a form, in which the degree of freedom of rotation corresponding to the restricted yaw operation in the leftward/rightward direction is secured when the manual manipulator part is used alone while the insertion of the locking shaft 1255 of the fifth joint 1223 that is a constant fixing shaft is released by the pushing of the spline 2223 of the driving part 2 when being mounted on the driving part 2, and through the mounting, the robot may be yawed through the mounting, and the yaw operation may be performed in an automatic manipulation mode.

Furthermore, referring to FIG. 11C, when the manipulator 1 is seated on the seating part 211, the rear end of the handle part 1231 may be rotated forward and a distal end of the front end of the handle part 1231 and the fixing tooth 1251 may be disengaged, and when the manipulator 1 is seated on the seating part 211, the handle part 1231 and the first joint 1211 may be rotated naturally, and thus, as described above, a pitch operation may be performed by rotating the handle part 1231.

Furthermore, the yaw operation manipulating part 22 may include yaw operation structures 221 and 222 that rotate the spline 223. The yaw operation structures 221 and 222 may include a motor 221, and a gear structure 222 that transmits a rotational force of the rotary shaft of the motor 221 to the spline 223. The gear structure 222 may include a first gear that is provided to surround the rotary shaft of the motor 221 and is rotated by rotation of the rotary shaft, a second gear that is coupled to a lower portion of the spline 223, and a third gear that transmits the rotation of the first gear to the second gear. One or more third gears may be provided. When the rotational force of the motor 212 is transmitted to the second gear through the first gear and the third gear and the second gear is rotated, the spline 223 may be rotated and the fifth joint 1223 may be rotated by the rotation of the spline 223. Furthermore, as the fifth joint 1223 is rotated, the operation module 11 may be rotated in the direction, in which the fifth joint 1223 has rotated, as described above.

Furthermore, referring to FIGS. 10A, 10B, 14 and 15, the robot may include a grip part 25 that fixes a position of the manipulator 1 seated on the seating part 211. The grip part 25 may grip the case 125 of the manipulator 1, and accordingly, the first joint 1211 or the fifth joint 1223 may be rotated while the case 125 is fixed so that a pitch operation, a yaw operation, or the like may be performed.

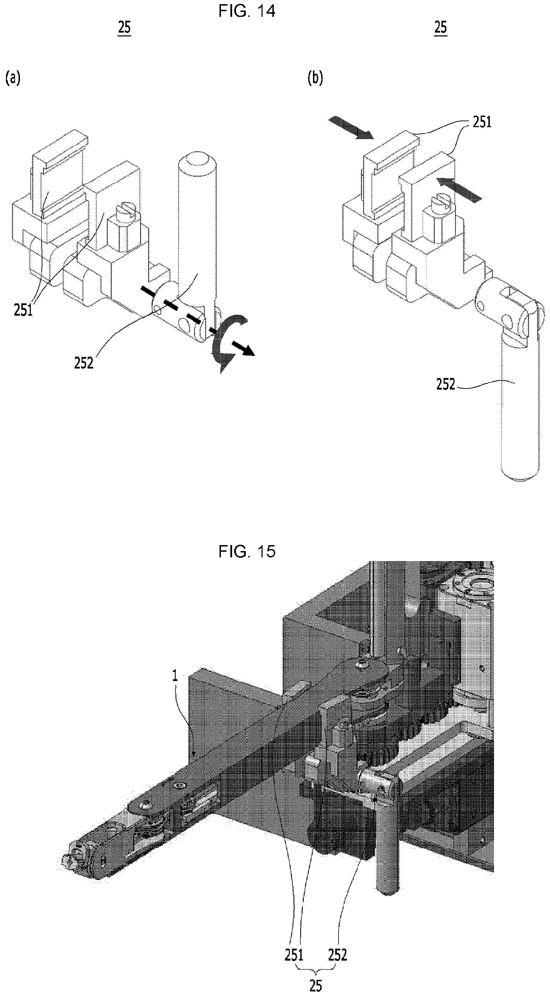

Referring to FIG. 10A, FIG. 10B, FIG. 14, and FIG. 15, the grip part 25 may include a pair of grip parts 251 that are disposed on the left and right sides while having an interval on a front side of the seating part 211, and the interval the grip unit 251 in the leftward/rightward direction may be adjusted. As the grip unit 251 becomes closer, the manipulator 1 located between the pair of grip units 251 may be gripped. Furthermore, as they become more distant, the grip of the manipulator 1 may be released. To this end, referring to FIGS. 14 and 15, the grip part 251 may include an interval adjusting unit 252 that adjusts an interval between the pair of grip parts 251.

As described above, when the manipulator 1 is fastened to the automatic driving part, the yaw axis, the pitch axis, and the roll axis are driven by a motor so that the driving part 2 automates the operation of the manipulator 1.

The robot may have characteristics of replacing a manual operation with a robot type manipulator in the form of using an actuator for assistance.

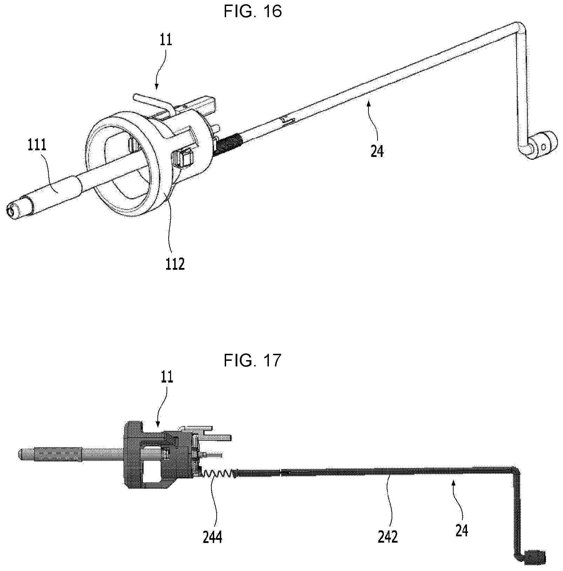

Furthermore, referring to FIGS. 16 and 17, the robot may include an automatic needle insertion part 24. The automatic needle insertion part 24 may move the needle 246 forward.

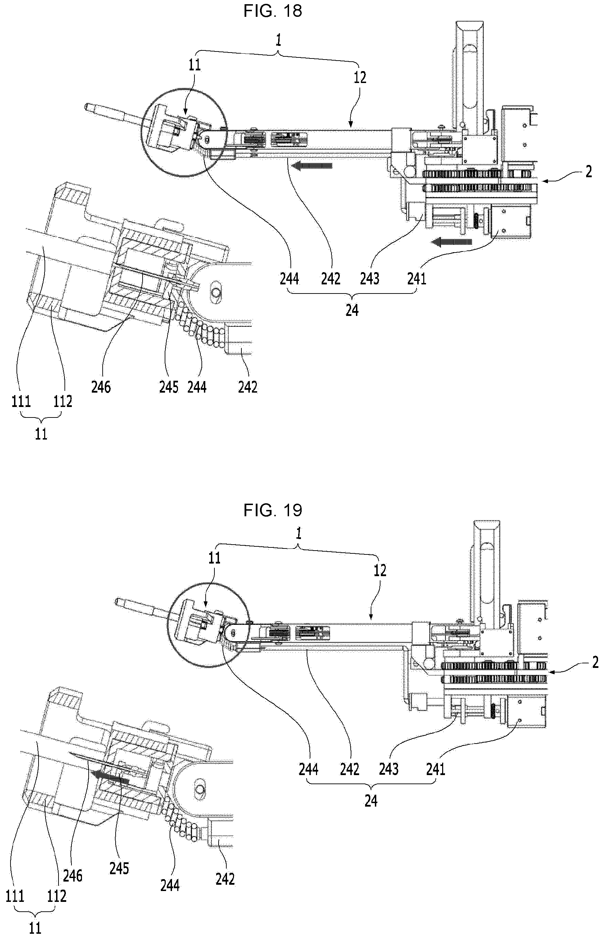

For example, referring to FIGS. 18 and 19, the automatic needle insertion part 24 may include a grip part 245 that is provided inside the cup 112 to grip the needle. Furthermore, the automatic needle insertion part 24 may include an additional connection member 244, a front end of which is coupled to the grip part 245 to extend rearward. Furthermore, the automatic needle insertion part 24 may include a connection member 242 that is provided to be movable in the forward/rearward direction in combination with the rear end of the additional connection member 244.

Furthermore, referring to FIGS. 18 and 19, the automatic needle insertion part 24 may include motor structures 241 and 243 that move the connection member 242 in the forward/rearward direction. For example, the motor structures 241 and 243 may include a motor 241 that provides movement power in the forward/rearward direction, and a linkage 243 that moves the connection member 242 in the forward/rearward direction by transmitting the movement power to the connection member 242. The needle 246 may be moved in the forward/rearward direction by the motor structures 241 and 243. Furthermore, the forward movement of the needle 246, that is, the operation of injecting the needle 246 into the uterus may be used when the operation module 11 is in a neutral state. The neutral state may mean the position of the operation module 11 when the angle of the operation module 11 in the upward/downward direction with respect to the forward/rearward direction corresponds to 0° (the same within an error range) and the angle in the leftward/rightward direction with respect to the forward/rearward direction corresponds to 0°.

Furthermore, the needle 246 may be connected to a drug injection module. Accordingly, the drug may be supplied to the needle 246 and the drug may be injected through the needle 246.

Conventionally, depending on the surgical situation, there are cases, in which it is necessary to inject the drug into the cervix, but to this end, there is a hassle of demounting the uterine manipulator from the uterus, injecting it with the cervix in the vagina, and then reinstalling the uterine manipulator. On the other hand, according to the present disclosure, a needle 246 that is connected to the drug injection module to receive a drug from the drug injection module and injecting the drug may be provided in the manipulator 1, so that the manipulator 1 may be inserted while being installed in the uterus, which solves the conventional troublesomeness.

Furthermore, referring to FIGS. 18 and 19, the additional connection member 244 may include an elastic member that allows a behavior in correspondence to the pitch operation and the yaw operation of the operation module 11. For example, the additional connection member 244 may be a spring. Accordingly, even in the pitch operation and the yaw operation of the operation module 11, a forward/rearward movement force may be transmitted to the grip part 245 while the additional connection member 244 is deformed and the connection state of the connection member 242 and the grip part 245 are maintained.

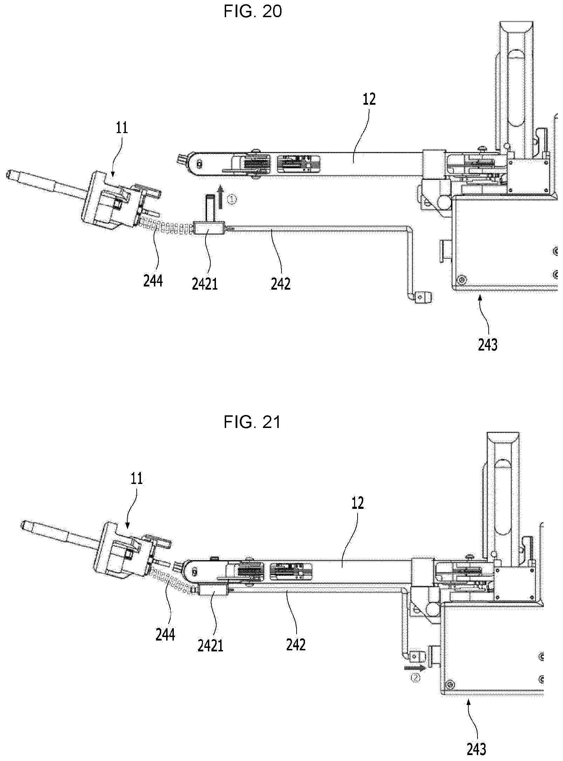

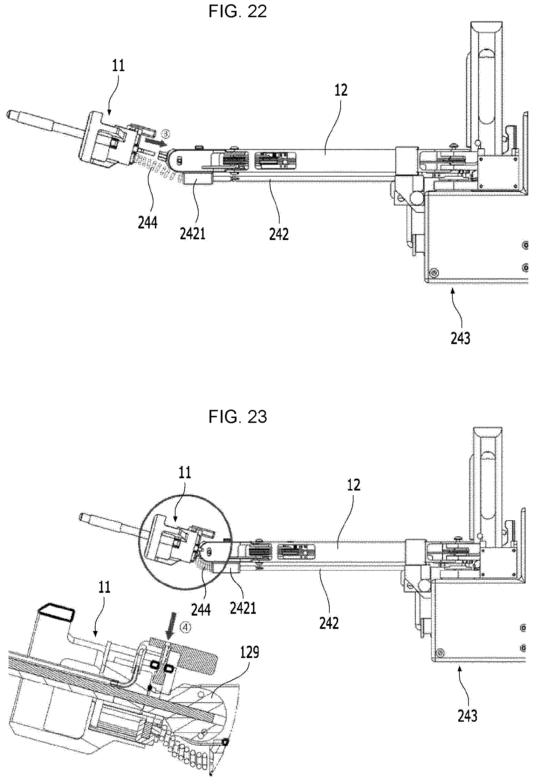

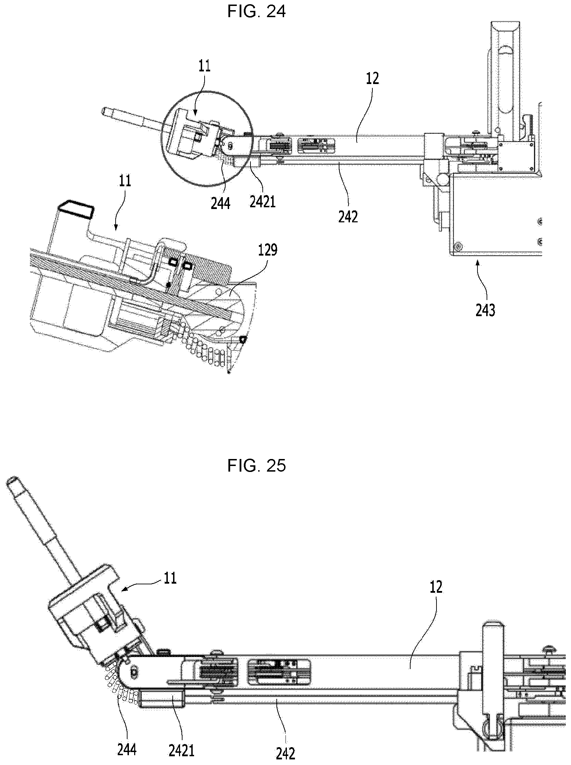

Meanwhile, the operation module 11 may be mounted on the operation structure 12 as illustrated in FIGS. 20 to 24. To this end, the front end of the connection member 242 may be provided with a boss 2421 that is inserted upward into the mounting part 1221 from a lower side, referring to FIG. 20, the boss 2421 may be fastened to the mounting part 1221, referring to FIGS. 21 to 23, a rear end of the end tip 111 may be inserted into and mounted on the end member 129, and referring to FIGS. 23 and 24, the mounting of the operation module 11 on the operation structure 12 may be completed by a separate fastening member.

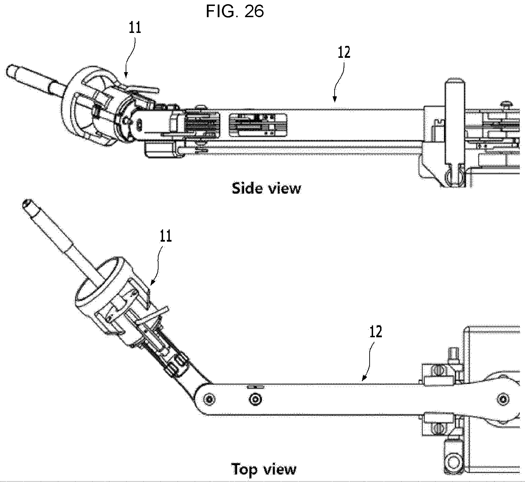

FIG. 25 illustrates a left side view of a part of the manipulator 1, and FIG. 26 illustrates a left side view and a plan view of a portion of the manipulator 1, in which the illustration of the additional connection member 244 is omitted.

Hereinafter, a uterine manipulator system (hereinafter, referred to as "the system") according to an embodiment of the present disclosure will be described. However, the system includes the robot and the manipulator 1 described above and shares the same or corresponding technical features and configurations to those of the robot and the manipulator 1. Accordingly, a repeated description of the description described in the robot and the manipulator 1 will be simplified or omitted, and the same reference numerals will be used for the same or similar configurations.

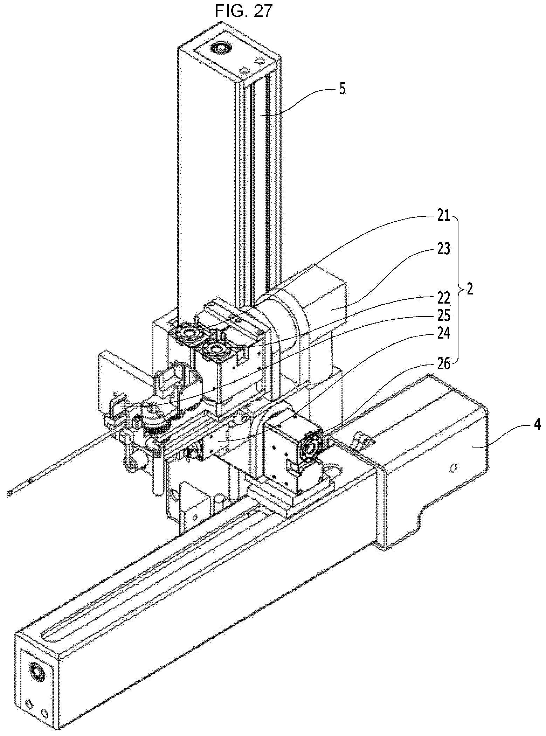

Referring to FIG. 27, this system includes the above-described robot.

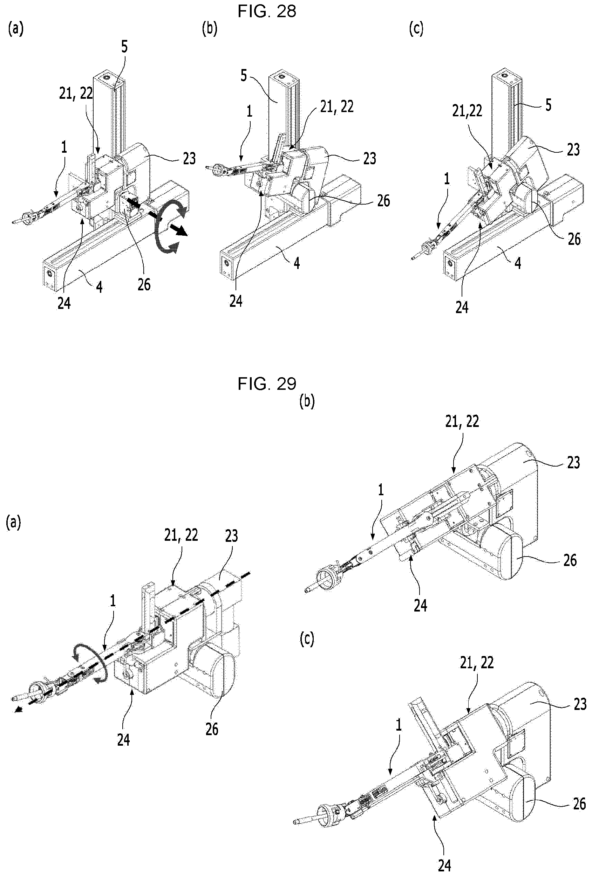

Furthermore, referring to FIGS. 27 to 28, the system includes an additional pitch operation part 26 that enables a pitch operation of rotating the end member 129 about a first axis of the robot such that a pitch in a direction which the end tip 111 faces is adjusted. Referring to FIG. 28, the pitch operation part 26 may adjust the pitch of the robot by rotating the robot while the leftward/rightward direction is taken as a rotation axis thereof.

Furthermore, referring to FIG. 27, the robot may include a roll operation part 23 that enables a roll operation of rotating the robot while the forward/rearward direction is taken as a rotation axis thereof. Referring to FIG. 29, the roll operation part 23 may implement a roll operation of the robot by rotating the robot while the forward/rearward direction is taken as a rotation axis thereof. The roll operation part 23 may include a motor, and the robot may be fastened to the rotary shaft of the motor.

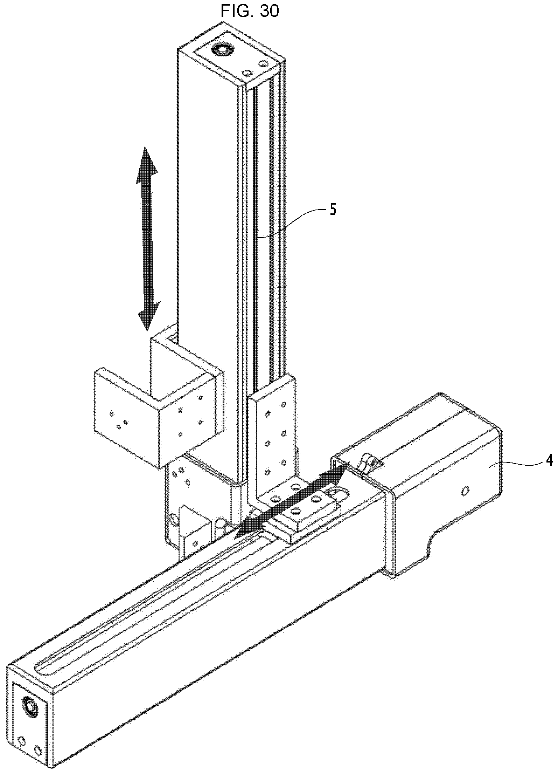

Furthermore, referring to FIGS. 27 and 30, the robot may include a first rail structure 5 that moves the robot upward and downward. The first rail structure 5 may include a first rail that extends upward and downward, and a guide part, by which the robot is on the first rail, and that allows the robot to move upward and downward along the first rail.

Furthermore, referring to FIGS. 27 and 30, the system may include a second rail structure 5 that moves the robot in the forward/rearward direction. The second rail structure 4 may include a second rail that extends in the forward/rearward direction, and a guide part, by which the robot is on the second rail, and that allows the robot to move in the forward/rearward direction along the second rail.

Furthermore, the system may include a controller that controls the robot, the additional pitch operation part 26, the roll operation part 23, the first rail structure 5, and the second rail structure 4.

According to the system, the manipulator 1 may enable an axial linear movement (an x-axis (forward/rearward direction) linear movement and a z-axis (upward/downward direction) linear movement), and enables a pitch movement, a yay movement, and a roll movement (a pitch rotation, a yaw rotation, and a roll rotation). That is, the manipulator 1 may enable a three degree of freedom of rotation, may enable a two degree of freedom of linear driving, and may enable a pitch rotation by the additional pitch operation part 26. Furthermore, for reference, the manipulator 1 may enable only a pitch movement during a manual manipulation while not being mounted on the driving part 2.

According to the conventional uterine manipulator robot system, a manipulation including a workspace outside the patient is performed, and an excessive movement may be required for manipulating the uterus, and thus, there is a concern that the uterus may be damaged during the manipulation, whereas it may be more advantageous in an aspect of stability during the surgery because the system performs a manipulation only on the cervix in all directions.

As described above, the manipulator 1, the robot, and the system, by which a master-slave manipulation (driving) system is constructed so that the stability and accuracy of the surgery may be secured by directly adjusting desired position and posture of the uterus, may be provided.

Furthermore, as described above, the manipulator 1, the robot, and the system, by which there is no need for a conventional surgical assistant to adjust the position or posture of the uterus, and the conventional surgical process (removal of the mounted tip/cup complex, injection of a drug, re-insertion of a tip/cup complex) may be omitted when an additional drug is needed in the cervix, may be provided.

Furthermore, the manipulator 1, the robot, and the system, by which an insertion depth of the drug and an amount of the injected drug may be quantified by utilizing the drug injection function so that the safety and accuracy of the surgery may be improved, may be provided. Furthermore, when it is necessary to inject an additional drug into the cervix, the existing surgical process may be omitted, and thus, the surgical time may be shortened.

## Claims

1. A uterine manipulator (1) comprising:
an operation module (11) including an end tip (111); and
an operation structure (12) including an end member (129) provided with the operation module (11) at a front end thereof, and a pitch operation part (121) allowing a pitch operation of rotating the end member (129) about a first axis such that a pitch in a direction which the end tip (111) faces is adjusted,
wherein the pitch operation part (121) includes:
a first joint (1211) disposed to be rotatable while an upward/downward direction is taken as a rotation axis thereof;
a second joint (1212) disposed on a front side of the first joint (1211), and disposed to be rotatable while the upward/downward direction is taken as a rotation axis thereof;
a first connection part (1213) configured to rotate the second joint (1212) by transmitting a rotational force of the first joint (1211) to the second joint (1212) when the first joint (1211) is rotated;
a third joint (1214) disposed on a front side of the second joint (1212), provided with the end member (129) at a front end thereof, and disposed to be rotatable while a leftward/rightward direction is taken as a rotation axis thereof; and
a second connection part (1215) configured to rotate the third joint (1214) by transmitting a rotational force of the second joint (1212) to the third joint (1214) when the second joint (1212) is rotated,
a yaw operation part (122) allowing a yaw operation about a second axis being perpendicular to the end member (129) such that a yaw in a direction, in which the end tip (111) faces, is adjusted,
**characterized in that** the yaw operation part (122) includes:
a mounting part (1221), on which the third joint (1214) is mounted;
a fourth joint (1222) disposed on a rear side of the mounting part (1221), and disposed to be rotatable while the upward/downward direction is taken as a rotation axis thereof;
a fifth joint (1223) disposed on a rear side of the fourth joint (1222), and disposed to be rotatable while the upward/downward direction is taken as a rotation axis thereof;
a first link part (1226) configured to transmit a rotational force of the fifth joint (1223) to the fourth joint (1222) when the fifth joint (1223) is rotated, or to transmit a rotational force of the fourth joint (1222) to the fifth joint (1223) when the fourth joint (1222) is rotated; and
a second link part (1227) configured to transmit the rotational force of the fourth joint (1222) to the mounting part (1221) when the fourth joint (1222) is rotated, or to rotate the mounting part (1221) in a rotational direction of the fourth joint (1222).

2. The uterine manipulator (1) of claim 1, wherein the first connection part (1213) is wires extending forward in two rows while surrounding at least a portion of the first joint (1211) and configured to surround at least a portion of the second joint (1212), and
wherein the pitch operation part (121) further includes:
a sixth joint (1216) provided between the first joint (1211) and the second joint (1212), and configured to guide an operation of the wire while supporting the wire.

3. The uterine manipulator (1) of claim 1, wherein the second connection part (1215) includes a left connection member (12152) extending forward from a left portion of the second joint (1212) and connected to a left portion of the third joint (1214), and a right connection member (12151) extending forward from a right portion of the second joint (1212) and connected to a right portion of the third joint (1214), and
wherein one of the left connection member (12152) and the right connection member (12151) extends from an upper portion of the second joint (1212) and is connected to an upper portion of the third joint (1214), and the other one extends from a lower portion of the second joint (1212) and is connected to a lower portion of the third joint (1214).

4. The uterine manipulator (1) of claim 1, wherein the operation structure (12) further includes:
a handle part (123) connected to the rotary shaft (1211a) of the first joint (1211), and configured to manipulate rotation of the first joint (1211).

5. The uterine manipulator (1) of claim 1, wherein the fifth joint (1223) is a pinion gear,
wherein the first link part (1226) includes two rows of first link members (12261, 12262) provided with rack gears (1226a) engaged with a left portion and a right portion of the fifth joint (1223) at rear ends thereof, respectively, provided with rack gears (1226a) at front ends thereof, respectively, and extending forward, and
wherein the fourth joint (1222) includes a left pinion gear (12222) engaged with the rack gear (1226a) of the front end of the left first link member, among the two rows of first link members (12261, 12262), and a right pinion gear (12221) engaged with the rack gear (1226a) of the front end of the right first link member, among the two rows of first link members (12261, 12262).

6. The uterine manipulator (1) of claim 5, wherein the pitch operation part (121) further includes a sixth joint (1216) provided between the first joint (1211) and the second joint (1212), and configured to guide an operation of the wire while supporting the wire, and
wherein one of the left pinion gear (12222) and the right pinion gear (12221) is provided at one of an upper portion and a lower portion of a rotary shaft (1216a) of the sixth joint (1216), and the other one of the left pinion gear (12222) and the right pinion gear (12221) is provided at the other one of the upper portion and the lower portion of the rotary shaft (1216a) of the sixth joint (1216).

7. The uterine manipulator (1) of claim 5, wherein the second link part (1227) includes a second upper link member (12271) extending from an upper portion of the sixth joint (1216) and connected to an upper portion of the mounting part (1221), and a second lower link member (12272) extending from a lower portion of the sixth joint (1216) and connected to a lower portion of the mounting part (1221).

8. A uterine manipulator robot comprising:
the uterine manipulator (1) of claim 1; and
a driving part (2) configured to drive the operation structure (12),
wherein the uterine manipulator (1) further includes:
a handle part (123) connected to the rotary shaft (1211a) of the first joint (1211), and configured to manipulate rotation of the first joint (1211) about a rotation axis thereof, and
wherein the driving part (2) includes:
a seating part (211), on which a rear end of the uterine manipulator (1) is seated; and
an automatic pitch manipulation part (21) configured to rotate the handle part (123) seated on the seating part (211).

9. The uterine manipulator robot of claim 8, wherein the manipulator (1) further includes a yaw operation part (122) allowing a yaw operation about a second axis being perpendicular to the first axis of the end member (129) such that a yaw in a direction, in which the end tip (111) faces, is adjusted. and
wherein the uterine manipulator robot further includes a yaw operation manipulating part (22).

10. The uterine manipulator robot of claim 9, wherein the yaw operation part (122) includes:
a mounting part (1221), on which the third joint (1214) is mounted;
a fourth joint (1222) disposed on a rear side of the mounting part (1221), and disposed to be rotatable while the upward/downward direction is taken as a rotation axis thereof;
a fifth joint (1223) disposed on a rear side of the fourth joint (1222), and disposed to be rotatable while the upward/downward direction is taken as a rotation axis thereof;
a locking shaft (1255) crossing the fifth joint (1223) upward and downward;
a first link part (1226) configured to transmit a rotational force of the fifth joint (1223) to the fourth joint (1222) when the fifth joint (1223) is rotated, or to transmit a rotational force of the fourth joint (1222) to the fifth joint (1223) when the fourth joint (1222) is rotated; and
a second link part (1227) configured to transmit the rotational force of the fourth joint (1222) to the mounting part (1221) when the fourth joint (1222) is rotated, or to rotate the mounting part (1221) in a rotational direction of the fourth joint (1222),
wherein the fifth joint (1223) is provided on a lower side of the first joint (1211), and the rotary shaft (1211a) of the first joint (1211) and the locking shaft (1255) of the fifth joint (1223) are connected to each other upward and downward, and
wherein the yaw operation manipulating part (22) includes:
a spline (223) protruding upward from a seating surface of the seating part (211), configured to move the locking shaft (1255) of the fifth joint (1223) upward when the manipulator (1) is seated on the seating part (211), demount the locking shaft (1255) from the fifth joint (1223), and replace the locking shaft (1255) of the fifth joint (1223); and
a yaw operation structure (221, 222) configured to rotate the spline (223).

11. The uterine manipulator robot of claim 8, wherein the operation module (11) further includes a cup (112) surrounding a rear end of an end tip (111), and a needle (246) provided inside the cup (112), and
wherein the uterine manipulator robot further includes an automatic needle insertion part (24) configured to move the needle (246) forward.

12. The uterine manipulator robot of claim 11, wherein the automatic needle insertion part (24) includes:
a grip part (245) provided in an interior of the cup (112) and configured to grip the needle (246);
an additional connection member (244), a front end of which is coupled to the grip part (245) and extends rearward;
a connection member (242) coupled to a rear end of the additional connection member (244) to be movable in a forward/rearward direction; and
a motor structure (241, 243) configured to move the connection member (242) in the forward/rearward direction.

13. The uterine manipulator robot of claim 12, wherein the additional connection member (244) includes an elastic member configured to behave in correspondence to a pitch operation and a yaw operation of the operation module (11).

## Patentansprüche

1. Uterusmanipulator (1), der aufweist:
ein Bedienmodul (11), das eine Endspitze (111) aufweist; und
eine Bedienstruktur (12), die ein Endelement (129) aufweist, das an seinem vorderem Ende mit dem Bedienmodul (11) versehen ist, und ein Nickbetätigungsteil (121) aufweist, der eine Nickbetätigung zum Drehen des Endelements (129) um eine erste Achse ermöglicht, sodass ein Nicken in einer Richtung, der die Endspitze (111) zugewandt ist, eingestellt wird,
wobei das Nickbetätigungsteil (121) aufweist:
ein erstes Gelenk (1211), das so angeordnet ist, dass es drehbar ist, wobei eine Aufwärts-/Abwärtsrichtung als dessen Drehachse dient;
ein zweites Gelenk (1212), das an einer Vorderseite des ersten Gelenks (1211) angeordnet und so ausgelegt ist, dass es drehbar ist, wobei die Aufwärts-/Abwärtsrichtung als seine Drehachse dient;
ein erstes Verbindungsteil (1213), das so ausgebildet ist, dass es das zweite Gelenk (1212) dreht, indem es eine Drehkraft des ersten Gelenks (1211) auf das zweite Gelenk (1212) überträgt, wenn das erste Gelenk (1211) gedreht wird;
ein drittes Gelenk (1214), das an einer Vorderseite des zweiten Gelenks (1212) angeordnet ist, an dessen vorderem Ende das Endelement (129) vorgesehen ist und das so angeordnet ist, dass es drehbar ist, wobei eine Links-/Rechtsrichtung als dessen Drehachse dient; und
ein zweites Verbindungsteil (1215), das so ausgebildet ist, dass es das dritte Gelenk (1214) dreht, indem es eine Drehkraft des zweiten Gelenks (1212) auf das dritte Gelenk (1214) überträgt, wenn das zweite Gelenk (1212) gedreht wird;
ein Gierbetätigungsteil (122), das eine Gierbetätigung um eine zweite Achse ermöglicht, die senkrecht zum Endelement (129) ist, sodass ein Gieren in einer Richtung, in der die Endspitze (111) zeigt, eingestellt wird,
**dadurch gekennzeichnet, dass** das Gierbetätigungsteil (122) aufweist:
ein Befestigungsteil (1221), an dem das dritte Gelenk (1214) befestigt ist;
ein viertes Gelenk (1222), das an einer Rückseite des ersten Befestigungsteils (1221) angeordnet und so ausgelegt ist, dass es drehbar ist, wobei die Aufwärts-/Abwärtsrichtung als seine Drehachse dient;
ein fünftes Gelenk (1223), das an einer Rückseite des vierten Gelenks (1222) angeordnet und so ausgelegt ist, dass es drehbar ist, wobei die Aufwärts-/Abwärtsrichtung als seine Drehachse dient;
ein erstes Verbindungsteil (1226), das so ausgebildet ist, dass es eine Drehkraft des fünften Gelenks (1223) auf das vierte Gelenk (1222) überträgt, wenn das fünfte Gelenk (1223) gedreht wird, oder eine Drehkraft des vierten Gelenks (1222) auf das fünfte Gelenk (1223) überträgt, wenn das vierte Gelenk (1222) gedreht wird; und
ein zweites Verbindungsteil (1227), das so ausgebildet ist, dass es die Drehkraft des vierten Gelenks (1222) auf das Befestigungsteil (1221) überträgt, wenn das vierte Gelenk (1222) gedreht wird, oder das Befestigungsteil (1221) in einer Drehrichtung des vierten Gelenks (1222) dreht.

2. Uterusmanipulator (1) nach Anspruch 1, wobei das erste Verbindungsteil (1213) aus Drähten besteht, die sich in zwei Reihen nach vorne erstrecken, dabei zumindest einen Teil des ersten Gelenks (1211) umschließen und so ausgebildet sind, dass sie zumindest einen Teil des zweiten Gelenks (1212) umschließen, und wobei das Nickbetätigungsteil (121) ferner aufweist:
ein sechstes Gelenk (1216), das zwischen dem ersten Gelenk (1211) und dem zweiten Gelenk (1212) angeordnet und so ausgebildet ist, dass es einen Betrieb des Drahtes führt und gleichzeitig den Draht stützt.

3. Uterusmanipulator (1) nach Anspruch 1, wobei das zweite Verbindungsteil (1215) ein linkes Verbindungselement (12152) aufweist, das sich von einem linken Abschnitt des zweiten Gelenks (1212) nach vorne erstreckt und mit einem linken Abschnitt des dritten Gelenks (1214) verbunden ist, und ein rechtes Verbindungselement (12151) aufweist, das sich von einem rechten Abschnitt des zweiten Gelenks (1212) nach vorne erstreckt und mit einem rechten Abschnitt des dritten Gelenks (1214) verbunden ist, und
wobei sich entweder das linke Verbindungselement (12152) oder das rechte Verbindungselement (12151) von einem oberen Abschnitt des zweiten Gelenks (1212) erstreckt und mit einem oberen Abschnitt des dritten Gelenks (1214) verbunden ist, und das andere sich von einem unteren Abschnitt des zweiten Gelenks (1212) erstreckt und mit einem unteren Abschnitt des dritten Gelenks (1214) verbunden ist.

4. Uterusmanipulator (1) nach Anspruch 1, wobei die Bedienstruktur (12) ferner aufweist:
ein Griffteil (123), das mit der Drehwelle (1211a) des ersten Gelenks (1211) verbunden und dazu ausgebildet ist, die Drehung des ersten Gelenks (1211) zu manipulieren.

5. Uterusmanipulator (1) nach Anspruch 1, wobei das fünfte Gelenk (1223) ein Ritzel ist,
wobei das erste Verbindungsteil (1226) zwei Reihen von ersten Verbindungsgliedern (12261, 12262) aufweist, die mit Zahnstangen (1226a) versehen sind, die an ihren hinteren Enden jeweils mit einem linken Abschnitt und einem rechten Abschnitt des fünften Gelenks (1223) in Eingriff stehen, an ihren vorderen Enden jeweils mit Zahnstangen (1226a) versehen sind und sich nach vorne erstrecken, und
wobei das vierte Gelenk (1222) ein linkes Ritzel (12222) aufweist, das mit der Zahnstange (1226a) am vorderen Ende des linken ersten Verbindungsglieds aus den beiden Reihen von ersten Verbindungsgliedern (12261, 12262) in Eingriff steht, und ein rechtes Ritzel (12221) aufweist, das mit der Zahnstange (1226a) am vorderen Ende des rechten ersten Verbindungsglieds aus den beiden Reihen von ersten Verbindungsgliedern (12261, 12262) in Eingriff steht.

6. Uterusmanipulator (1) nach Anspruch 5, wobei das Nickbetätigungsteil (121) ferner ein sechstes Gelenk (1216) aufweist, das zwischen dem ersten Gelenk (1211) und dem zweiten Gelenk (1212) vorgesehen und so ausgebildet ist, dass es einen Betrieb des Drahtes führt und gleichzeitig den Draht stützt, und
wobei entweder das linke Ritzel (12222) oder das rechte Ritzel (12221) an entweder dem oberen Abschnitt oder dem unteren Abschnitt einer Drehwelle (1216a) des sechsten Gelenks (1216) vorgesehen ist, und das jeweils andere des linken Ritzels (12222) und des rechten Ritzels (12221) am jeweils anderen der beiden des oberen Abschnitts und des unteren Abschnitts der Drehwelle (1216a) des sechsten Gelenks (1216) angeordnet ist.

7. Uterusmanipulator (1) nach Anspruch 5, wobei das zweite Verbindungsteil (1227) ein zweites oberes Verbindungsglied (12271) aufweist, das sich von einem oberen Abschnitt des sechsten Gelenks (1216) erstreckt und mit einem oberen Abschnitt des Befestigungsteils (1221) verbunden ist, und ein zweites unteres Verbindungselement (12272) aufweist, das sich von einem unteren Abschnitt des sechsten Gelenks (1216) erstreckt und mit einem unteren Abschnitt des Befestigungsteils (1221) verbunden ist.

8. Uterusmanipulatorroboter, der aufweist:
den Uterusmanipulator (1) nach Anspruch 1; und
ein Antriebsteil (2), das zum Antreiben der Bedienstruktur (12) ausgebildet ist,
wobei der Uterusmanipulator (1) ferner aufweist:
ein Griffteil (123), das mit der Drehwelle (1211a) des ersten Gelenks (1211) verbunden und dazu ausgebildet ist, die Drehung des ersten Gelenks (1211) um dessen Drehachse zu manipulieren, und
wobei das Antriebsteil (2) aufweist:
ein Auflageteil (211), auf dem ein hinteres Ende des Uterusmanipulators (1) aufliegt; und
ein automatisches Nickmanipulationsteil (21), das so ausgebildet ist, dass es den auf dem Auflageteil (211) aufliegenden Griffteil (123) dreht.

9. Uterusmanipulatorroboter nach Anspruch 8, wobei der Manipulator (1) ferner ein Gierbetätigungsteil (122) aufweist, das eine Gierbetätigung um eine zweite Achse ermöglicht, die senkrecht zur ersten Achse des Endelements (129) ist, sodass ein Gieren in einer Richtung, in der die Endspitze (111) zeigt, eingestellt wird, und
wobei der Uterusmanipulatorroboter ferner ein Gierbetätigungsmanipulationsteil (22) aufweist.

10. Uterusmanipulatorroboter nach Anspruch 9, wobei das Gierbetätigungsteil (122) aufweist:
ein Befestigungsteil (1221), an dem das dritte Gelenk (1214) befestigt ist;
ein viertes Gelenk (1222), das an einer Rückseite des ersten Befestigungsteils (1221) angeordnet und so ausgelegt ist, dass es drehbar ist, wobei die Aufwärts-/Abwärtsrichtung als seine Drehachse dient;
ein fünftes Gelenk (1223), das an einer Rückseite des vierten Gelenks (1222) angeordnet und so ausgelegt ist, dass es drehbar ist, wobei die Aufwärts-/Abwärtsrichtung als seine Drehachse dient;
eine Verriegelungswelle (1255), die das fünfte Gelenk (1223) aufwärts und abwärts durchquert;
ein erstes Verbindungsteil (1226), das so ausgebildet ist, dass es eine Drehkraft des fünften Gelenks (1223) auf das vierte Gelenk (1222) überträgt, wenn das fünfte Gelenk (1223) gedreht wird, oder eine Drehkraft des vierten Gelenks (1222) auf das fünfte Gelenk (1223) überträgt, wenn das vierte Gelenk (1222) gedreht wird; und
ein zweites Verbindungsteil (1227), das so ausgebildet ist, dass es die Drehkraft des vierten Gelenks (1222) auf das Befestigungsteil (1221) überträgt, wenn das vierte Gelenk (1222) gedreht wird, oder das Befestigungsteil (1221) in einer Drehrichtung des vierten Gelenks (1222) dreht,
wobei das fünfte Gelenk (1223) an einer Unterseite des ersten Gelenks (1211) vorgesehen ist und die Drehwelle (1211a) des ersten Gelenks (1211) und die Verriegelungswelle (1255) des fünften Gelenks (1223) aufwärts und abwärts miteinander verbunden sind, und
wobei der Gierbetätigungsmanipulationsteil (22) aufweist:
eine Keilwelle (223), die von einer Auflagefläche des Auflageteils (211) nach oben vorsteht und so ausgebildet ist, dass sie die Verriegelungswelle (1255) des fünften Gelenks (1223) nach oben bewegt, wenn der Manipulator (1) auf dem Auflageteil (211) aufliegt, die Verriegelungswelle (1255) von dem fünften Gelenk (1223) demontiert und die Verriegelungswelle (1255) des fünften Gelenks (1223) austauscht; und
eine Gierbetätigungsstruktur (221, 222), die so ausgebildet ist, dass sie die Keilwelle (223) dreht.

11. Uterusmanipulatorroboter nach Anspruch 8, wobei das Bedienmodul (11) ferner einen Becher (112), der ein hinteres Ende einer Endspitze (111) umgibt, und eine Nadel (246) aufweist, die innerhalb der Manschette (112) vorgesehen ist, und
wobei der Uterusmanipulatorroboter ferner ein automatisches Nadeleinführteil (24) aufweist, das so ausgebildet ist, dass es die Nadel (246) nach vorne bewegt.

12. Uterusmanipulatorroboter nach Anspruch 11, wobei das automatische Nadeleinführteil (24) aufweist:
ein Griffteil (245), das im Inneren des Bechers (112) vorgesehen und so ausgebildet ist, dass es die Nadel (246) greift;
ein zusätzliches Verbindungselement (244), dessen vorderes Ende mit dem Griffteil (245) verbunden ist und sich nach hinten erstreckt;
ein Verbindungselement (242), das mit einem hinteren Ende des zusätzlichen Verbindungselements (244) verbunden ist, um in Vorwärts-/Rückwärtsrichtung bewegbar zu sein; und eine Motorstruktur (241, 243), die so ausgebildet ist, dass sie das Verbindungselement (242) in Vorwärts-/Rückwärtsrichtung bewegt.

13. Uterusmanipulatorroboter nach Anspruch 12, wobei das zusätzliche Verbindungselement (244) ein elastisches Element aufweist, das so ausgebildet ist, dass es sich entsprechend eines Nickvorgangs und eines Giervorgangs des Bedienmoduls (11) verhält.

## Revendications

1. Manipulateur utérin (1), comprenant :
un module de mise en œuvre (11) comprenant une pointe d'extrémité (111) ; et
une structure de mise en œuvre (12) comprenant un élément d'extrémité (129) doté du module de mise en œuvre (11) au niveau de son extrémité avant, et une partie de mise en œuvre d'inclinaison longitudinale (121) permettant une mise en œuvre d'inclinaison longitudinale de rotation de l'élément d'extrémité (129) autour d'un premier axe de façon à régler une inclinaison longitudinale dans une direction dans laquelle est orientée la pointe d'extrémité,
dans lequel la partie de mise en œuvre d'inclinaison longitudinale (121) comprend :
une première articulation (1211) disposée de façon à pouvoir tourner en prenant une direction haut/bas en tant qu'axe de rotation de cette dernière ;
une deuxième articulation (1212) disposée d'un côté avant de la première articulation (1211), et disposée de façon à pouvoir tourner en prenant la direction haut/bas en tant qu'axe de rotation de cette dernière ;
une première partie d'accouplement (1213) configurée pour entraîner en rotation la deuxième articulation (1212) par une transmission d'une force de rotation de la première articulation (1211) à la deuxième articulation (1212) lors de l'entraînement en rotation de la première articulation (1211) ;
une troisième articulation (1214) disposée d'un côté avant de la deuxième articulation (1212), dotée de l'élément d'extrémité (129) au niveau de son extrémité avant, et disposée de façon à pouvoir tourner en prenant une direction gauche/droite en tant qu'axe de rotation de cette dernière ; et
une seconde partie d'accouplement (1215) configurée pour entraîner en rotation la troisième articulation (1214) par une transmission d'une force de rotation de la deuxième articulation (1212) à la troisième articulation (1214) lors de l'entraînement en rotation de la deuxième articulation (1212),
une partie de mise en œuvre de lacet (122) permettant une mise en œuvre de lacet autour d'un second axe qui est perpendiculaire à l'élément d'extrémité (129) de façon à régler un lacet dans une direction dans laquelle est orientée la pointe d'extrémité (111),
**caractérisé en ce que** la partie de mise en œuvre de lacet (122) comprend :
une partie de montage (1221), sur laquelle est montée la troisième articulation (1214) ;
une quatrième articulation (1222) disposée d'un côté arrière de la partie de montage (1221), et disposée de façon à pouvoir tourner en prenant la direction haut/bas en tant qu'axe de rotation de cette dernière ;
une cinquième articulation (1223) disposée d'un côté arrière de la quatrième articulation (1222), et disposée de façon à pouvoir tourner en prenant la direction haut/bas en tant qu'axe de rotation de cette dernière ;
une première partie biellette de liaison (1226) configurée pour transmettre une force de rotation de la cinquième articulation (1223) à la quatrième articulation (1222) lors de l'entraînement en rotation de la cinquième articulation (1223), ou pour transmettre une force de rotation de la quatrième articulation (1222) à la cinquième articulation (1223) lors de l'entraînement en rotation de la quatrième articulation (1222) ; et
une seconde partie biellette de liaison (1227) configurée pour transmettre la force de rotation de la quatrième articulation (1222) à la partie de montage (1221) lors de l'entraînement en rotation de la quatrième articulation (1222), ou pour entraîner en rotation la partie de montage (1221) dans un sens de rotation de la quatrième articulation (1222).

2. Manipulateur utérin (1) selon la revendication 1, dans lequel la première partie d'accouplement (1213) est constituée de fils s'étendant vers l'avant sur deux rangées tout en entourant au moins une partie de la première articulation (1211) et configurés pour entourer au moins une partie de la deuxième articulation (1212), et
dans lequel la parie de mise en œuvre d'inclinaison longitudinale (121) comprend en outre :
une sixième articulation (1216) disposée entre la première articulation (1211) et la deuxième articulation (1212), et configurée pour guider une mise en œuvre du fil tout en supportant le fil.

3. Manipulateur utérin (1) selon la revendication 1, dans lequel la seconde partie d'accouplement (1215) comprend un élément d'accouplement gauche (12152) s'étendant vers l'avant d'une partie gauche de la deuxième articulation (1212) et accouplé à une partie gauche de la troisième articulation (1214), et un élément d'accouplement droit (12151) s'étendant vers l'avant d'une partie droite de la deuxième articulation (1212) et accouplé à une partie droite de la troisième articulation (1214), et
dans lequel l'un de l'élément d'accouplement gauche (12152) et de l'élément d'accouplement droit (12151) s'étend à partir d'une partie supérieure de la deuxième articulation (1212) et est accouplé à une partie supérieure, et l'autre s'étend à partir d'une partie inférieure de la deuxième articulation (1212) et est accouplé à une partie inférieure de la troisième articulation (1214).

4. Manipulateur utérin (1) selon la revendication 1, dans lequel la structure de mise en œuvre (12) comprend en outre :
une partie poignée (123) reliée à l'arbre rotatif (1211a) de la première articulation (1211), et configurée pour manipuler une rotation de la première articulation (1211).

5. Manipulateur utérin (1) selon la revendication 1, dans lequel la cinquième articulation (1223) est un pignon d'engrenage,
dans lequel la première partie biellette de liaison (1226) comprend deux rangées de premiers éléments de biellette de liaison (12261, 12262) pourvus d'engrenages à crémaillère (1226a) en prise respectivement avec une partie gauche et avec une partie droite de la cinquième articulation (1223) au niveau de leurs extrémités arrière, pourvus respectivement d'engrenages à crémaillère (1226a) au niveau de leurs extrémités avant, et s'étendant vers l'avant, et
dans lequel la quatrième articulation (1222) comprend un pignon d'engrenage gauche (12222) en prise avec l'engrenage à crémaillère (1226a) de l'extrémité avant du premier élément de biellette de liaison gauche, entre les deux rangées de premiers éléments de biellette de liaison (12261, 12262), et un pignon d'engrenage droit (12221) en prise avec l'engrenage à crémaillère (1226a) de l'extrémité avant du premier élément de biellette de liaison droit, entre les deux rangées de premiers éléments de biellette de liaison (12261, 12262).

6. Manipulateur utérin (1) selon la revendication 5, dans lequel la partie de mise en œuvre d'inclinaison longitudinale (121) comprend en outre une sixième articulation (1216) disposée entre la première articulation (1211) et la deuxième articulation (1212), et configurée pour guider une mise en œuvre du fil tout en supportant le fil, et
dans lequel l'un du pignon d'engrenage gauche (12222) et du pignon d'engrenage droit (12221) est disposé au niveau de l'une d'une partie supérieure et d'une partie inférieure d'un arbre rotatif (1216a) de la sixième articulation (1216), et l'autre du pignon d'engrenage gauche (12222) et du pignon d'engrenage droit (12221) est disposé au niveau de l'autre de la partie supérieure et de la partie inférieure de l'arbre rotatif (1216a) de la sixième articulation (1216).

7. Manipulateur utérin (1) selon la revendication 5, dans lequel la seconde partie biellette de liaison (1227) comprend un second élément de biellette de liaison supérieur (12271) s'étendant à partir d'une partie supérieure de la sixième articulation (1216) et relié à une partie supérieure de la partie de montage (1221), et un second élément de biellette de liaison inférieur (12272) s'étendant à partir d'une partie inférieure de la sixième articulation (1216) et relié à une partie inférieure de la partie de montage (1221).

8. Robot manipulateur utérin, comprenant :
le manipulateur utérin (1) selon la revendication 1 ; et
une partie d'entraînement (2) configurée pour entraîner la structure de mise en œuvre (12),
dans lequel le manipulateur utérin (1) comprend en outre :
une partie poignée (123) reliée à l'arbre rotatif (1211a) de la première articulation (1211), et configurée pour manipuler une rotation de la première articulation (1211) autour de son axe de rotation, et
dans lequel la partie d'entraînement (2) comprend :
une partie d'appui (211) sur laquelle est appuyé une extrémité arrière du manipulateur utérin (1) ; et
une partie de manipulation d'inclinaison longitudinale automatique (21) configurée pour faire tourner la partie poignée (123) appuyée sur la partie d'appui (211).

9. Robot manipulateur utérin selon la revendication 8, dans lequel le manipulateur (1) comprend en outre une partie de mise en œuvre de lacet (122) permettant une mise en œuvre de lacet autour d'un second axe qui est perpendiculaire au premier axe de l'élément d'extrémité (129) de façon à régler un lacet dans une direction dans laquelle est orientée la pointe d'extrémité (111), et
dans lequel le robot manipulateur utérin comprend en outre une partie de manipulation de mise en œuvre de lacet (22).

10. Robot manipulateur utérin selon la revendication 9, dans lequel la partie de mise en œuvre de lacet (122) comprend :
une partie de montage (1221), sur laquelle est montée la troisième articulation (1214) ;
une quatrième articulation (1222) disposée d'un côté arrière de la partie de montage (1221), et disposée de façon à pouvoir tourner en prenant la direction haut/bas en tant qu'axe de rotation de cette dernière ;
une cinquième articulation (1223) disposée d'un côté arrière de la quatrième articulation (1222), et disposée de façon à pouvoir tourner en prenant la direction haut/bas en tant qu'axe de rotation de cette dernière ;
un arbre de verrouillage (1255) traversant la cinquième articulation (1223) vers le haut et vers le bas ;
une première partie biellette de liaison (1226) configurée pour transmettre une force de rotation de la cinquième articulation (1223) à la quatrième articulation (1222) lors de l'entraînement en rotation de la cinquième articulation (1223), ou pour transmettre une force de rotation de la quatrième articulation (1222) à la cinquième articulation (1223) lors de l'entraînement en rotation de la quatrième articulation (1222) ; et
une seconde partie biellette de liaison (1227) configurée pour transmettre la force de rotation de la quatrième articulation (1222) à la partie de montage (1221) lors de l'entraînement en rotation de la quatrième articulation (1222), ou pour entraîner en rotation la partie de montage (1221) dans un sens de rotation de la quatrième articulation (1222),
dans lequel la cinquième articulation (1223) est disposée d'un côté inférieur de la première articulation (1211), et l'arbre rotatif (1211a) de la première articulation (1211) et l'arbre de verrouillage (1255) de la cinquième articulation (1223) sont liés l'un à l'autre vers le haut et vers le bas, et
dans lequel la partie de manipulation de mise en œuvre de lacet (22) comprend :
une clavette (223) en saillie vers le haut à partir d'une surface d'appui de la partie d'appui (211), configurée pour déplacer l'arbre de verrouillage (1255) de la cinquième articulation (1223) vers le haut lorsque la manipulateur (1) est appuyé sur la partie d'appui (211), pour démonter l'arbre de verrouillage (1255) de la cinquième articulation (1223), et pour remplacer l'arbre de verrouillage (1255) de la cinquième articulation (1223) ; et
une structure de mise en œuvre de lacet (221, 222) configurée pour entraîner en rotation la clavette (223).

11. Robot manipulateur utérin selon la revendication 8, dans lequel le module de mise en œuvre (11) comprend en outre une coupelle (112) entourant une extrémité arrière d'une pointe d'extrémité (111), et une aiguille (246) disposée à l'intérieur de la coupelle (112), et
dans lequel le robot manipulateur utérin comprend en outre une partie d'insertion d'aiguille automatique (24) configurée pour déplacer l'aiguille (246) vers l'avant.

12. Robot manipulateur utérin selon la revendication 11, dans lequel la partie d'insertion d'aiguille automatique (24) comprend :
une partie de saisie (245) disposée à l'intérieur de la coupelle (112) et configurée pour saisir l'aiguille (246) ;
un élément d'accouplement supplémentaire (244), dont une extrémité avant est accouplée à la partie de saisie (245) et s'étend vers l'arrière ;
un élément d'accouplement (242) accouplé à une extrémité arrière de l'élément d'accouplement supplémentaire (244) pour pouvoir être déplacé dans une direction avant/arrière ; et
une structure de moteur (241, 243) configurée pour déplacer l'élément d'accouplement (242) dans la direction avant/arrière.

13. Robot manipulateur utérin selon la revendication 12, dans lequel l'élément d'accouplement supplémentaire (244) comprend un élément élastique configuré pour se comporter en correspondance avec une mise en œuvre d'inclinaison longitudinale et avec une mise en œuvre de lacet du module de mise en œuvre (11).
